# EUROPEAN PATENT SPECIFICATION

(11) **EP 2 152 726 B1**
(45) Date of publication and mention of the grant of the patent: **26.11.2014**
(21) Application number: 08733530.3
(22) Date of filing: 30.04.2008
(51) Int. Cl.: C07K 7/08

(54) **ANALGESIC COMPOUNDS**
ANALGETISCHE VERBINDUNGEN
COMPOSES ANALGESIQUES

(30) Priority: 01.05.2007 US 915247 P
(43) Date of publication of application: 17.02.2010
(73) Proprietor: Fundação de Amparo à Pesquisa do Estado de São Paulo - FAPESP, 05468-901 São Paulo (BR); Biolab Sanus Farmacêutica Ltda., 06767-220, Taboão da Serra, São Paulo (BR); Cury, Yara, 05661-020 Sao Paulo (BR)
(72) Inventor: CURY, Yara, 05661-020 São Paulo -Sp (BR); PICOLO, Gisele, 03411-120 São Paulo - Sp (BR); KONNO, Katsuhiro, Santo Domingo (DO)
(74) Representative: Kling, Simone
(86) International application number: PCT/BR2008/000127
(87) International publication number: WO 2008/131508

(56) References cited:
- WO-A2-2005/107357
- US-A- 5 232 911
- ALLAN, L. ET AL: "The complete sequence of the acidic subunit from Mojave toxin determined by Edman degradation and mass spectrometry" BIOCHIMICA ET BIOPHYSICA ACTA, vol. 1037, 1990, pages 413-421, XP002577748
- FAURE G. ET AL.: 'Multiplicity of acidic subunit isoforms of crotoxin, the phospholipase A2 neurotoxin from Crotalus durissus terrificus venom, results from posttranslational modifications' BIOCHEMISTRY vol. 30, no. 32, 13 August 1991, pages 8074 - 8083, XP002476828
- AIRD S.D. ET AL.: 'Rattlesnake presynaptic neurotoxins: primary structure and evolutionary origin of the acidic subunit' BIOCHEMISTRY vol. 24, no. 25, 03 December 1985, pages 7054 - 7058, XP002476827

## Description

### BACKGROUND

By some estimates, pain affects at least 30% of individuals at some moment of their lives and for 10% to 40% of these individuals, pain lasts for more than one day.

The incidence of chronic pain in the world oscillates from 7% and 40% of the population. About 50% to 60% of individuals who suffer from chronic pain become partially or totally and temporarily or permanently incapacitated by the pain.

### SUMMARY

In general, the disclosure features novel compounds (e.g., peptides) that are useful in the treatment of pain. The compounds are useful in treating both chronic and acute pain. The peptides can act as analgesics and/or anti-nociceptive agents.

The present invention concerns an isolated peptide that comprises an amino acid sequence that is at least 90% identical to the amino acid sequence: pGlu-Phe-Ser-Pro-Glu-Asn-Ala-Gln-Gly-Glu-Ser-Gln-Pro-Ala (SEQ ID NO: 2 ), with the proviso that the peptide does not comprise the amino acid sequence SEQ ID NO: 3 or SEQ ID NO: 5 and wherein amino acid residues R7 and R14 are not linked by an intramolecular disulfide bridge.

The present invention also concerns a pharmaceutical composition comprising said peptide.

The present invention also concerns said pharmaceutical composition for use for treating or preventing painful conditions or conditions mediated by opioid receptors in a subject.

The present invention further concerns said pharmaceutical composition for use for treating or preventing pain in a subject.

The present invention also concerns said pharmaceutical composition for use for causing analgesia in a subject.

The present invention also relates to a process for producing a peptide comprising SEQ ID NO: 2, wherein the process comprises peptide synthesis in solid phase.

The application describes an isolated peptide that includes the amino acid sequence:
R1-R2-Ser-R4-R5-R6-R7-R8-Gly-R10-Ser-R12-Pro-R14 (SEQ ID NO: 1)
wherein:
R1 is pyroglutarnate,
R2 is Phe or Trp or Tyr or Leu or Thr,
R4 is Pro or Arg,
R5 is GIx or Asx or Gly,
R6 is Asn or Gln or Leu,
R7 is any amino acid,
R8 is any amino acid except Lys,
R10 is Glx or Asx,
R12 is GIx or Lys, and
R14 is any amino acid,
with the proviso that if R7 is Cys, R14 is not Cys and if R14 is Cys, R7 is not Cys. In some embodiments, R8 is Glx. In some embodiments, the peptide is capable of causing an analgesic or an anti-nociceptive effect in a subject.

Is also described, the peptide which consists of the amino acid sequence:
R1-R2-Ser-R4-R5-R6-R7-R8-Gly-R10-Ser-R12-Pro-R14 (SEQ ID NO:1)
wherein:
R1 is pyroglutamate,
R2 is Phe or Trp or Tyr or Leu or Thr'
R4 is Pro or Arg,
R5 is Glx or Asx or Gly,
R6 is Asn or Gln or Leu,
R7 is any amino acid,
R8 is any amino acid except Lys,
R10 is Glx or Asx,
R12 is Glx or Lys, and
R14 is any amino acid,
with the proviso that if R7 is Cys, R14 is not Cys and if R14 is Cys, R7 is not Cys. In some embodiments, R8 is Glx.

In some embodiments, includes the amino acid sequence of SEQ ID NO:2: pGlu-Phe-Ser-Pro-Glu-Asn-Ala-Gln-Gly-Glu-Ser-Gln-Pro-Ala.

In some embodiments, the peptide consists of the amino acid sequence of SEQ ID NO:2. A peptide consisting of SEQ ID NO:2 is referred to herein as the "7A14A peptide."

The application also describes an isolated peptide that includes an amino acid sequence that is at least 70% (e.g., 75%, 80%, 82%, 85%, 86%, 87%, 88%, 89%, 90%, 91%, 92%, 93%, 94%, 95%, 96%, 97%, 98%, 99%) identical to the amino acid sequence of SEQ ID NO:2, wherein the peptide is capable of causing an analgesic or an anti-nociceptive effect in a subject.

The application aslo describes an isolated peptide that includes an amino acid sequence that is at least 70% (e.g., 75%, 80%, 82%, 85%, 86%, 87%, 88%, 89%, 90%,91%, 92%, 93%, 94%, 95%, 96%, 97%, 98%, 99%) identical to the amino acid sequence of SEQ ID NO:2, wherein the amino acid sequence is not SEQ ID NO:3 or SEQ ID NO:5, and the peptide is capable of causing an analgesic or an anti-nociceptive effect in a subject.

A peptide consisting of amino acid sequence pGlu-Phe-Ser-Pro-Glu-Asn-Cys-Gln-Gly-Glu-Ser-Gln-Pro-Cys (SEQ ID NO:3) is referred to herein as the "ENPAK peptide." A peptide consisting of amino acid sequence pGlu-Phe-Ser-Pro-Glu-Asn-Cys-Gln-Gly-Glu-Ser-Lys-Pro-Cys (SEQ ID NO:5) is referred to herein as the "ENPAK analog."

The application also describes an isolated peptide that includes an amino acid sequence that is at least 70% (e.g., 75%, 80%, 82%, 85%, 86%, 87%, 88%, 89%, 90%, 91 %, 92%, 93%, 94%, 95%, 96%, 97%, 98%, 99%) identical to the amino acid sequence of SEQ ID NO:2, wherein R7 and R14 amino acids are not linked by an intramolecular disulfide bridge, and the peptide is capable of causing an analgesic or an anti-nociceptive effect in a subject.

Is also described the peptide which consists of an amino acid sequence that is at least 70%(e.g.,75%, 80%, 82%, 85%, 86%, 87%, 88%, 89%, 90%, 91 %,92%,93%,94%,95%, 96%, 97%, 98%, 99%) identical to the amino acid sequence of SEQ ID NO:2.

Is also described the peptide which consists of an amino acid sequence that is at least 70% (e.g., 75%, 80%, 82%, 85%, 86%, 87%, 88%, 89%, 90%, 91%, 92%, 93%, 94%, 95%, 96%,97%,98%,99%) identical to the amino acid sequence of SEQ ID NO:2, and the peptide is not SEQ ID NO:3 or SEQ ID NO:5.

Is also described the peptide which consists of an amino acid sequence that is at least 70% (e.g., 75%,80%,82%,85%,86%,87%,88%,89%,90%,91%,92%, 93%, 94%, 95%, 96%, 97%, 98%, 99%) identical to the amino acid sequence of SEQ ID NO:2, and wherein R7 and R14 amino acids are not linked by an intramolecular disulfide bridge.

The application describes an isolated peptide that includes an amino acid sequence that differs from the amino acid sequence of SEQ ID NO:2 or SEQ ID NO:3 or SEQ ID NO:5 by one, two, three, four, five, six, seven, eight, nine, ten, eleven, twelve, thirteen, or fourteen amino acid substitutions, additions, or deletions, wherein the peptide is capable of causing an analgesic or an anti-nociceptive effect in a subject.

As disclosed herein, the amino acid sequence may differ from the amino acid sequence of SEQ ID NO:2 or SEQ ID NO:3 or SEQ ID NO:5 by one, two, three, four, five, six, or seven conservative amino acid substitutions.

Is also described the peptide which consists of an amino acid sequence that differs from the amino acid sequence of SEQ ID NO:2 or SEQ ID NO:3 or SEQ ID NO:5 by one, two, three, four, five, six, seven, eight, nine, ten, eleven, twelve, thirteen, or fourteen amino acid substitutions, additions, or deletions. As described herein, the amino acid sequence may differ from the amino acid sequence of SEQ ID NO:2 or SEQ ID NO:3 or SEQ ID NO:5 by one, two, three, four, five, six, or seven conservative amino acid substitutions.

In another aspect, the disclosure provides a pharmaceutical composition that includes a peptide of the invention (e.g., in combination with one or more pharmaceutically acceptable excipients).

In some embodiments, the peptide includes the amino acid sequence of SEQ ID NO:2.

In some embodiments, the peptide consists of the amino acid sequence of SEQ ID NO:2. In some embodiments, the pharmaceutical composition is an oral dosage form (e.g., a tablet, a capsule, a caplet, a pill, a powder, a drop, a suspension, a solution, a paste, a gel).

In some embodiments, the pharmaceutical composition is formulated for enteral, parenteral, or topical administration.

In some embodiments, the pharmaceutical composition is formulated for enteral (e.g., oral) administration.

In some embodiments, the pharmaceutical composition is formulated for parenteral (e.g., intravenous injection or infusion (IV), intraarterial injection, subcutaneous injection (SC), intraperitoneally (IP), intracardiac injection, intraosseous infusion, intradermal injection, intraperitoneal infusion or injection, intravitreal injection, intramuscular injection, intrathecal injection, intra-articular injection, or epidural) administration.

In some embodiments, the pharmaceutical composition is formulated for topical administration.

In another aspect, the disclosure provides a method of treating or preventing pain in a subject. The method includes administering a pharmaceutical composition described herein (e.g., a pharmaceutical composition that contains a peptide of the invention) to the subject.

In some embodiments, the peptide includes the amino acid sequence of SEQ ID NO: 1 or SEQ ID NO:2.

In some embodiments, the peptide consists of the amino acid sequence of SEQ ID NO: 1 or SEQ ID NO:2.

In some embodiments, the pain is acute or chronic.

In some embodiments, the pain is nociceptive.

In some embodiments, the pain is somatic, visceral, or neuropathic.

In some embodiments, the pain is cancer pain.

In some embodiments, the pain is selected from the group consisting of: neoplasia-associated pains, fibromyalgia, dental pain, dysmenorrhea, renal pain, menstrual pain, biliary colic pain, joint pain, arthritis, intra-ocular hypertension, post-arthroscopy pain, post-laparoscopy gynecological pain, pain produced by percutaneous nephrolithotomy, radical retropubic post-prostatectomy pain, post-thoracotomy pain, post-tonsillectomy pain, post-hysterectomy pain, cesarean post-operation pain, and pain associated with: burns, cocaine dependence, opioid dependence, cellular proliferation, small cell pulmonary carcinoma, depression, psychosis, inflammation, conditions associated with an increase of angiogenesis, wounds, coronary ischemic disease, Parkinson's disease, dyskinesia, hepatic encephalopathy, cognitive disease, Alzheimer's disease, itch due to hepatic cholestasis, and hyperinsulinemia in women.

In some embodiments, the pain is neuropathic pain selected from the group consisting of trigeminal neuralgia, sympathetic dystrophy, post-herpetic neuralgia, phantom limb pain, post cerebralvascular accident, and diabetic neuropathy.

In some embodiments, pain is arthritis, e.g., the arthritis is rheumatoid arthritis or degenerative arthritis.

In some embodiments, the pharmaceutical compositionn is administered in combination with another treatment for pain (e.g., another treatment described herein).

In some aspects, the disclosure provides a method of causing an anti-nociceptive effect in a subject. The method includes administering a pharmaceutical composition described herein (e.g., a pharmaceutical composition that contains a peptide of the invention) to the subject.

In some embodiments, the peptide includes the amino acid sequence of SEQ ID NO:2.

In some embodiments, the peptide consists of the amino acid sequence of SEQ ID NO:2.

In some embodiments, the peptide acts on an opioid receptor (e.g., a kappa or delta opioid receptor).

In some embodiments, the peptide acts on the opioid receptor directly.

In other embodiments, the peptide acts on the opioid receptor indirectly.

In some embodiments, the peptide is an opioid receptor agonist or partial agonist. Drugs (e.g., peptides, e.g., the peptides described herein) that bind to physiological receptors and mimic the effects of the endogenous regulatory compounds are termed agonists. Drugs that are only partly as effective as agonists are termed partial agonists.

In some embodiments, the anti-nociceptive effect lasts up to 1,2,3,4, 5, 6, 7, or 8 days.

In some embodiments, the pharmaceutical composition is administered in combination with another treatment that is capable of causing an anti-nociceptive effect (e.g., another treatment described herein).

In another aspect, the disclosure provides a process for producing a peptide that contains SEQ ID NO:2. The process includes chemical synthesis of the peptide, which comprises solid phase synthesis.

The application also describes a recombinant DNA molecule that contains a DNA sequence which encodes a peptide that includes or consists of the amino acid sequence of SEQ ID NO: 1 (or SEQ ID NO:2); or the complementary strand thereof.

The application also describes a prokaryotic or eukaryotic host cell transformed with a vector which contains a DNA sequence that encodes a peptide that includes or consists of the amino acid sequence of SEQ ID NO:1 (or SEQ ID NO:2).

The application also describes a method for producing a peptide that contains or consists of the amino acid sequence of SEQ ID NO: 1 (or SEQ ID NO:2). The method includes the step of culturing a prokaryotic or eukaryotic host cell described herein, e.g., under conditions that allow (or induce) the expression of the DNA sequence contained therein.

In some embodiments, the method further includes purifying the peptide from the cell or cell culture medium.

The application describes an isolated peptide comprising, consisting essentially of, or consisting of the amino acid sequence pGlu-Phe-Ser-Pro-Glu-Asn-Cys-Gln-Gly-Glu-Ser-Gln-Pro-Cys (SEQ ID NO:3), wherein no intramolecular disulfide bridge is formed between the two Cys residues.

The application describes a pharmaceutical composition comprising, consisting essentially of, or consisting of the peptide of SEQ ID NO:3, wherein no intramolecular disulfide bridge is formed between the two Cys residues, e.g., with one or more pharmaceutically-acceptable excipients. In some embodiments, the pharmaceutical composition is an oral dosage form (e.g., a tablet, a capsule, a caplet, a pill, a powder, a drop, a suspension, a solution, a paste, a gel). In some embodiments, the pharmaceutical composition is formulated for enteral, parenteral, or topical administration. In some embodiments, the pharmaceutical composition is formulated for enteral (e.g., oral) administration. In some embodiments, the pharmaceutical composition is formulated for parenteral (e.g., intravenous injection or infusion (IV), intraarterial injection, subcutaneous injection (SC), intraperitoneally (IP), intracardiac injection, intraosseous infusion, intradermal injection, intraperitoneal infusion or injection, intravitreal injection, intramuscular injection, intrathecal injection, intra-articular injection, or epidural) administration. In some embodiments, the pharmaceutical composition is formulated for topical administration.

The application describes a method of treating or preventing pain in a subject, the method includes administering the pharmaceutical composition comprising, consisting essentially of, or consisting of the peptide of SEQ ID NO:3, wherein no intramolecular disulfide bridge is formed between the two Cys residues. In some embodiments, the pain is acute pain. In some embodiments, the pain is chronic pain. In some embodiments, the pain is selected from the group consisting of: cancer related pain, neuropathic pain, neoplasia-associated pains, fibromyalgia, dental pain, dysmenorrhea, renal, menstrual pain, biliary colic pain, joint pain, arthritis, intra-ocular hypertension, post-arthroscopy pain, post-Iaparoscopy gynecological pain, pain produced by percutaneous nephrolithotomy, radical retropubic post-prostatectomy pain, post-thoracotomy pain, post-tonsillectomy pain in pediatric subjects, post-hysterectomy pain, cesarean post-operation pain, and pain associated with: burns, cocaine dependence, opioid dependence, cellular proliferation, small cell pulmonary carcinoma, depression, psychosis, inflammation, conditions associated with an increase of angiogenesis, wounds, coronary ischemic disease, Parkinson's disease, dyskinesia, hepatic encephalopathy, cognitive disease, Alzheimer's disease, itch due to hepatic cholestasis, and hyperinsulinemia in women. In some embodiments, the pain is neuropathic pain selected from the group consisting of trigeminal neuralgia, sympathetic dystrophy, post-herpetic neuralgia, phantom limb pain, post cerebralvascular accident, and diabetic neuropathy. In some embodiments, the arthritis is rheumatoid arthritis or degenerative arthritis. In some embodiments, the pharmaceutical composition is administered in combination with another treatment (e.g., another treatment described herein) for pain.

The application describes a method of causing an anti-nociceptive effect in a subject, the method includes administering the pharmaceutical composition comprising, consisting essentially of, or consisting of the peptide of SEQ ID NO:3, wherein no intramolecular disulfide bridge is formed between the two Cys residues. In some embodiments, the peptide acts on an opioid receptor (e.g., a kappa or delta opioid receptor). In some embodiments, the peptide acts on the opioid receptor directly. In some embodiments, the peptide acts on the opioid receptor indirectly. In some embodiments, the peptide is an opioid receptor agonist or partial agonist. In some embodiments, the anti-nociceptive effect lasts up to 1,2, 3,4, 5, 6, 7, or 8 days. In some embodiments, the pharmaceutical composition is administered in combination with another treatment (e.g., another treatment described herein) that is capable of causing an anti-nociceptive effect.

The application describes a process for producing a peptide that contains SEQ ID NO:3, wherein no intramolecular disulfide bridge is formed between the two Cys residues. The process includes chemical synthesis of the peptide.

In some embodiments, the chemical synthesis comprises solid phase synthesis.

The application describes a recombinant DNA molecule that contains a DNA sequence which encodes a peptide that includes or consists of the amino acid sequence of SEQ ID NO:3, wherein no intramolecular disulfide bridge is formed between the two Cys residues; or the complementary strand thereof.

The application describes a prokaryotic or eukaryotic host cell transformed with a vector which contains a DNA sequence that encodes a peptide that includes or consists of the amino acid sequence of SEQ ID NO:3, wherein no intramolecular disulfide bridge is formed between the two Cys residues. The application also describes a method for producing a peptide that contains or consists of the amino acid sequence of SEQ ID NO:3, wherein no intramolecular disulfide bridge is formed between the two Cys residues. The method includes the step of culturing a prokaryotic or eukaryotic host cell described herein, e.g., under conditions that allow (or induce) the expression of the DNA sequence contained therein.

In some embodiments, the method further includes purifying the peptide from the cell or cell culture medium.

The application describes an isolated peptide that includes the amino acid sequence:
R1-R2-Ser-R4-R5-R6-Cys-R8-Gly-R10-Ser-R12-Pro-Cys (SEQ ID NO:4)
wherein:
RI is pyroglutamate,
R2 is Phe or Trp or Tyr or Leu or Thr,
R4 is Pro or Arg,
R5 is Glx or Asx or Gly,
R6 is Asn or GIn or Leu,
R8 is any amino acid except Lys,
RIO is Glx or Asx, and
RI2 is Glx or Lys,
wherein no intramolecular disulfide bridge is formed between the two Cys residues at positions R7 and R14, with the proviso that the amino acid sequence is other than SEQ ID NO:3. In some embodiments, the peptide is capable of causing an analgesic or an anti-nociceptive effect in a subject. In some embodiments, R8 is Glx.

Is described, the peptide which consists of the amino acid sequence:
RI-R2-Ser-R4-R5-R6-Cys-R8-Gly-RIO-Ser-RI2-Pro-Cys (SEQ ID NO:4)
wherein:
RI is pyroglutamate,
R2 is Phe or Trp or Tyr or Leu or Thr,
R4 is Pro or Arg,
R5 is Glx or Asx or Gly,
R6 is Asn or GIn or Leu,
R8 is any amino acid except Lys,
R10 is Glx or Asx, and
R12 is Glx or Lys,
wherein no intramolecular disulfide bridge is formed between the two Cys residues at positions R7 and R14, with the proviso that the amino acid sequence is other than SEQ ID NO:3. In some embodiments, the peptide is capable of causing an analgesic or an anti¬nociceptive effect in a subject. In some embodiments, R8 is Glx.

The application describes a pharmaceutical composition comprising, consisting essentially of, or consisting of the peptide of SEQ ID NO:4, wherein no intramolecular disulfide bridge is formed between the two Cys residues at postions R7 and R14, with the proviso that the amino acid sequence is other than SEQ ID NO:3, e.g., with one or more pharmaceutically-acceptable excipients. In some embodiments, the pharmaceutical composition is an oral dosage form (e.g., a tablet, a capsule, a caplet, a pill, a powder, a drop, a suspension, a solution, a paste, a gel). In some embodiments, the pharmaceutical composition is formulated for enteral, parenteral, or topical administration. In some embodiments, the pharmaceutical composition is formulated for enteral (e.g., oral) administration. In some embodiments, the pharmaceutical composition is formulated for parenteral (e.g., intravenous injection or infusion (IV), intraarterial injection, subcutaneous injection (SC), intraperitoneally (IP), intracardiac injection, intraosseous infusion, intradermal injection, intraperitoneal infusion or injection, intravitreal injection, intramuscular injection, intrathecal injection, intra-articular injection, or epidural) administration. In some embodiments, the pharmaceutical composition is formulated for topical administration.

The application describes a method of treating or preventing pain in a subject, the method includes administering the pharmaceutical composition comprising, consisting essentially of, or consisting of the peptide of SEQ ID NO:4, wherein no intramolecular disulfide bridge is formed between the two Cys residues at positions R7 and R14, with the proviso that the amino acid sequence is other than SEQ ID NO:3. In some embodiments, the pain is acute pain. In some embodiments, the pain is chronic pain. In some embodiments, the pain is selected from the group consisting of: cancer related pain, neuropathic pain, neoplasia-associated pains, fibromyalgia, dental pain, dysmenorrhea, renal, menstrual pain, biliary colic pain, joint pain, arthritis, intra-ocular hypertension, post-arthroscopy pain, post¬laparoscopy gynecological pain, pain produced by percutaneous nephrolithotomy, radical retropubic post-prostatectomy pain, post-thoracotomy pain, post-tonsillectomy pain in pediatric subjects, post-hysterectomy pain, cesarean post-operation pain, and pain associated with: burns, cocaine dependence, opioid dependence, cellular proliferation, small cell pulmonary carcinoma, depression, psychosis, inflammation, conditions associated with an increase of angiogenesis, wounds, coronary ischemic disease, Parkinson's disease, dyskinesia, hepatic encephalopathy, cognitive disease, Alzheimer's disease, itch due to hepatic cholestasis, and hyperinsulinemia in women. In some embodiments, the pain is neuropathic pain selected from the group consisting of trigeminal neuralgia, sympathetic dystrophy, post-herpetic neuralgia, phantom limb pain, post cerebralvascular accident, and diabetic neuropathy. In some embodiments, the arthritis is rheumatoid arthritis or degenerative arthritis. In some embodiments, the pharmaceutical composition is administered in combination with another treatment (e.g., another treatment described herein) for pain.

The application describes a method of causing an anti-nociceptive effect in a subject, the method includes administering the pharmaceutical composition comprising, consisting essentially of, or consisting of the peptide of SEQ ID NO:4, wherein no intramolecular disulfide bridge is formed between the two Cys residues at positions R7 and R14, with the proviso that the amino acid sequence is other than SEQ ID NO:3. In some embodiments, the peptide acts on an opioid receptor (e.g., a kappa or delta opioid receptor). In some embodiments, the peptide acts on the opioid receptor directly. In some embodiments, the peptide acts on the opioid receptor indirectly. In some embodiments, the peptide is an opioid receptor agonist or partial agonist. In some embodiments, the anti-nociceptive effect lasts up to I, 2, 3, 4, 5, 6, 7, or 8 days. In some embodiments, the pharmaceutical composition is administered in combination with another treatment (e.g., another treatment described herein) that is capable of causing an anti-nociceptive effect.

The application describes a process for producing a peptide that contains SEQ ID NO:4, wherein no intramolecular disulfide bridge is formed between the two Cys residues at positions R7 and R14, with the proviso that the amino acid sequence is other than SEQ ID NO:3. The process includes chemical synthesis of the peptide.

In some embodiments, the chemical synthesis comprises solid phase synthesis.

The application describes a recombinant DNA molecule that contains a DNA sequence which encodes a peptide that includes or consists of the amino acid sequence of SEQ ID NO:4, wherein no intramolecular disulfide bridge is formed between the two Cys residues at positions R7 and R14, with the proviso that the amino acid sequence is other than SEQ ID NO:3; or the complementary strand thereof.

The application describes a prokaryotic or eukaryotic host cell transformed with a vector which contains a DNA sequence that encodes a peptide that includes or consists of the amino acid sequence of SEQ ID NO:4, wherein no intramolecular disulfide bridge is formed between the two Cys residues at positions R7 and R14, with the proviso that the amino acid sequence is other than SEQ ID NO:3.

The application describes a method for producing a peptide that contains or consists of the amino acid sequence of SEQ ID NO:4, wherein no intramolecular disulfide bridge is formed between the two Cys residues at positions R7 and R14, with the proviso that the amino acid sequence is other than SEQ ID NO:3. The method includes the step of culturing a prokaryotic or eukaryotic host cell described herein, e.g., under conditions that allow (or induce) the expression of the DNA sequence contained therein.

In some embodiments, the method further includes purifying the peptide from the cell or cell culture medium.

The application describes a compound described herein (e.g., a peptide described herein, e.g., in a pharmaceutical composition, e.g., a pharmaceutical composition described herein) for use in therapy.

The application describes the use of a compound described herein (e.g., a peptide described herein, e.g., in a pharmaceutical composition, e.g., a pharmaceutical composition described herein) for the preparation of a medicament for the treatment of pain in a subject (e.g., mammal, e.g., human).

The analgesic effect of a peptide described herein (e.g., in a pharmaceutical composition, e.g., a pharmaceutical composition described herein) can last, e.g., for about 2, 4, 8, 12,16,20,24,28,32,36,40,44,48,52,56,60,64,68, 72, 76,80,84,88,92,96,100,104,108, 112, 116, 120, 124, 128, 132, 136, 140, 144, 148, 152, 156, 160, 164, 168, 172, 176, 180, 184, 188, 192, 196, 200, 204, 208, 212, 216, 220, 224, 228, 232, 236, 240, 244, 248,252, 256, 260, 264, 268, 272, 276, 280, 284, 288, 292, 296, 300 hours. For example, the analgesic effect can last up to about 0.5, 1, 2, 3, 4, 5, 6, 7, 8, 9, 10, 11, 12, 13, or 14 or more days.

The term "analgesic effect" refers to the ability to bring about analgesia, e.g., a neurologic or pharmacologic state in which painful stimuli (e.g., pain), even if still perceived, they are no longer painful.

The anti-nociceptive effect of a peptide described herein (e.g., in a pharmaceutical composition, e.g., a pharmaceutical composition described herein) can last, e.g., for about 2, 4, 8, 12, 16, 20, 24, 28, 32, 36, 40, 44, 48, 52, 56, 60, 64, 68, 72, 76, 80, 84, 88, 92, 96, 100, 104, 108, 112, 116, 120, 124, 128, 132, 136, 140, 144, 148, 152, 156, 160, 164, 168, 172, 176, 180, 184, 188,192,196,200,204,208,212,216,220,224,228, 232, 236, 240, 244, 248, 252, 256, 260, 264, 268, 272, 276, 280, 284, 288, 292, 296, 300 hours. For example, the anti-nociceptive effect can last up to about 0.5, 1, 2, 3, 4, 5, 6, 7, 8, 9, 10, 11, 12, 13, or 14 or more days.

The term "anti-nociceptive effect" refers to the blocking of a pain signal transmission after nociceptor activation.

The term "treating" refers to administering a therapy (e.g., peptide described herein, e.g., in a pharmaceutical composition, e.g., a pharmaceutical composition described herein) in an amount, manner, and/or mode effective to improve or prevent a condition, symptom, or parameter associated with a disorder (e.g., pain), to prevent onset, progression, or exacerbation of the disorder (including pain caused by a disorder (e.g., cancer) or event, (e.g., surgery or accident)), or to decrease the perception of pain (e.g., discomfort) experienced by a subject to either a statistically significant degree or to a degree detectable to one skilled in the art. Treating a subject includes pain management. Accordingly, treating can achieve therapeutic and/or prophylactic benefits. An effective amount, manner, or mode can vary depending on the subject and may be tailored to the subject.

The details of one or more embodiments of the invention are set forth in the accompanying drawings and the description below.

### DESCRIPTION OF DRAWINGS

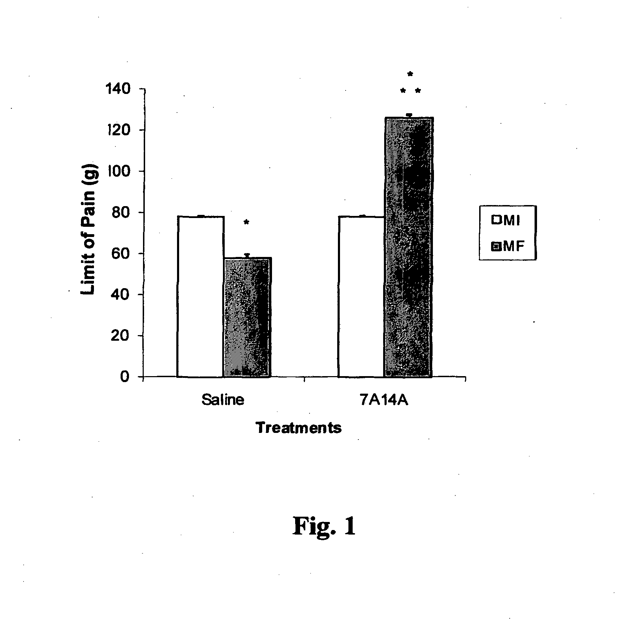

FIG. 1 is a bar graph depicting the pain threshold of rats treated with saline or the 7A14A peptide in a hyperalgesia model. MI: the pain threshold prior to treatment; MF: the pain threshold after administration of the treatment. For the evaluation of pain sensitivity, a rat paw pressure test was utilized. The limit of pain, represented by the force (in grams) necessary for withdrawal of the paw, was determined before (initial measure) and 3h after (final measure) the intraplantar injection of prostaglandin E2 (100 ng/paw). The peptide (5 µg/kg) or saline (control group) were orally administered, immediately before the harmful agent. The data represent the average ± e.p.m. of 5 animals per group. *p<0.05 by comparison with the initial measure and **p<0,05 for comparison with the control group.

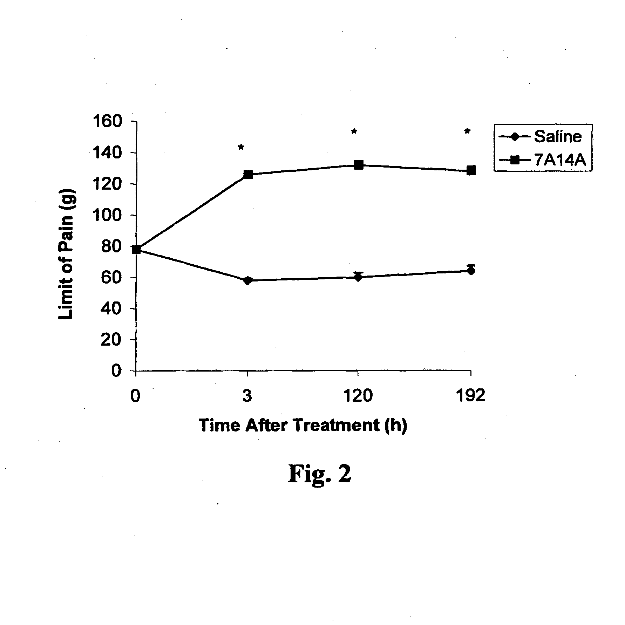

FIG. 2 is a line graph depicting the duration of the anti-nociceptive effect caused by the 7A14A peptide in a hyperalgesia model in rats. For the evaluation of pain sensitivity, a rat paw pressure test was utilized. The limit of pain, represented by the force (in grams) necessary for withdrawal of the paw, was determined before (time 0) and 3, 120, and 192h (final measure) after the oral treatment, with the synthetic peptide (5 µg/kg) or saline (control group). Prostaglandin (100 ng/paw), utilized as an hyperalgesic agent, was injected 3 h before each final measure. The data represent the average ± e.p.m. of 6 animals per group. *p<0.05 for comparison with the control group.

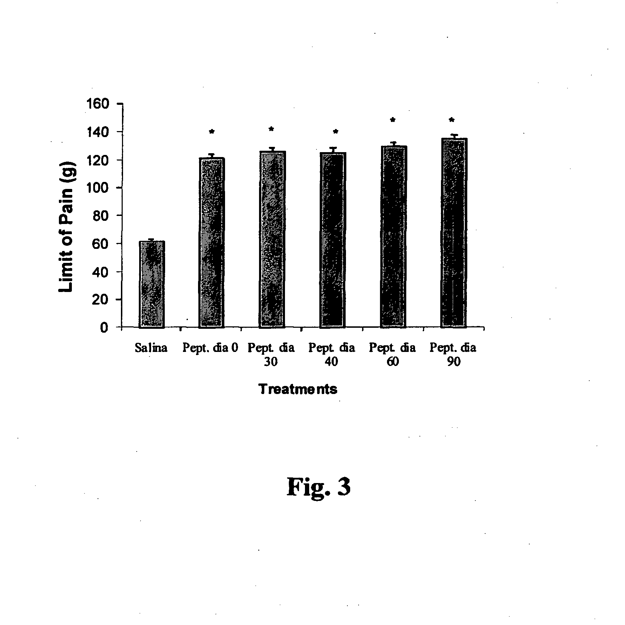

FIG 3 is a bar graph depicting the pain threshold of rats treated with saline or the 7A14A peptide in a hyperalgesia model. The 7A14A peptide was diluted and then tested in the model at 0, 30, 40, 60, or 90 days after dilution. For the evaluation of pain sensitivity, a rat paw pressure test was utilized. The limit of pain, represented by the force (in grams) necessary for withdrawal of the paw, was determined 3h after the intraplantar injection of prostaglandin E₂ (100 ng/paw). The peptide (5 µg/kg), diluted at the moment of use or after 30, 40, 60 e 90 dias (days) of dilution, or salina (saline) (control group) were orally administered, immediately before the harmful agent. The data represent the average ± e.p.m. of 5 animals per group. *p<0.05 for comparison with the initial measure, and **p<0,05 for comparison with the control group.

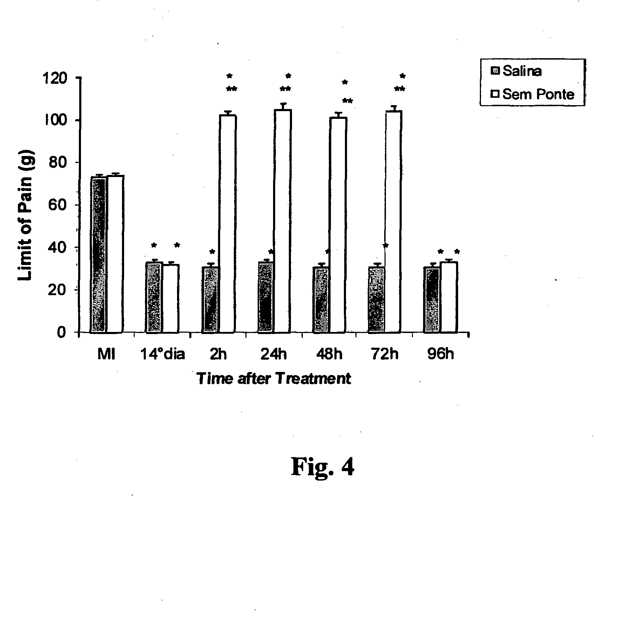

FIG 4 is a bar graph depicting the pain threshold in a hyperalgesia model in rats treated with saline or the 7A14A peptide. For the induction of neuropathic pain, surgery was performed to cronically constrict the sciatic nerve, according to the method described by Bennett & Xie (1988). For evaulation of hyperalgesia, a rat paw pressure test was utilized (Analgesy-Meter Ugo Basile®, Itália; Randall & Sellito (1957). The animals were orally treated with salina (saline) (control) or sem ponte (peptide 7A14A (1µg/kg)). The paw pressure test was applied before treatment (initial measure), on dia (day) 14, and at 24, 48, 72 and 96h after oral administration of saline or peptide.

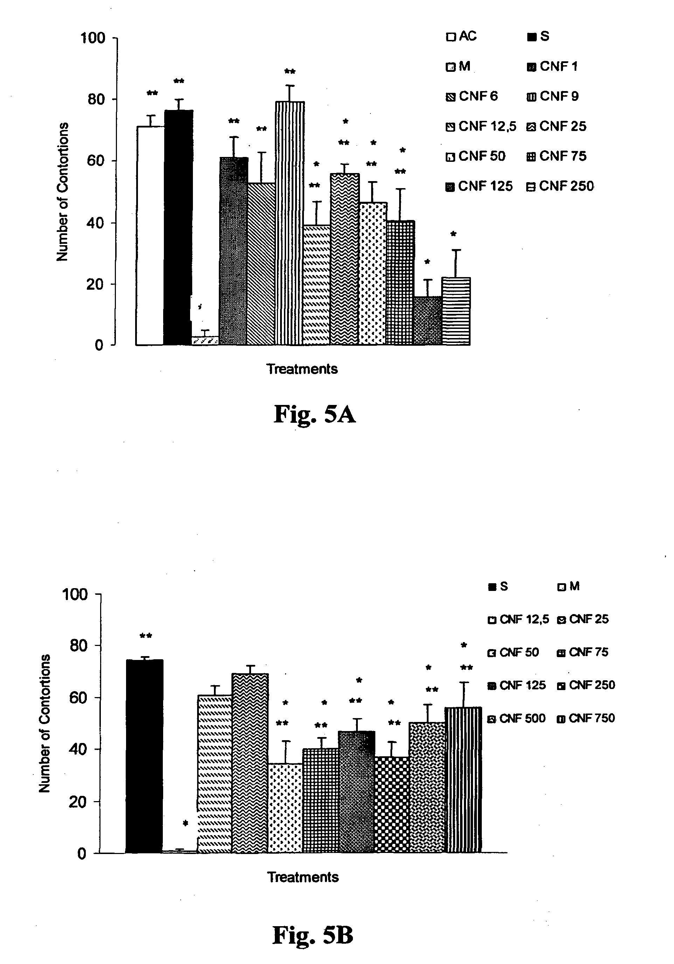

FIGS. 5A and 5B are bar graphs depicting the anti-nociceptice effect caused by ENPAK and 7A14A peptides in a visceral pain model in mice.

### DETAILED DESCRIPTION

### Overview

The disclosure features novel peptides that are useful for the treatment of pain, e.g., acute or chronic pain. The peptides can decrease pain sensitivity and have an analgesic effect. In some embodiments, the peptides have an anti-nociceptive effect. The peptides can induce analgesia, e.g., by activating opioid receptors. The peptides can cause long lasting analgesic and/or anti-nociceptive effects. Also described herein are methods of using such peptides (e.g., for the treatment of pain), methods of making such peptides, and kits and compositions containing such peptides.

### Pain and Nociception

The International Association for the Study of Pain (IASP) defines pain as "an unpleasant sensory and emotional experience which we primarily associate with tissue damage or describe in terms of such damage, or both." This definition recognizes that pain is a combined sensory, emotional, and cognitive phenomenon and that physical pathology does not need to be present for a patient to experience pain. Conceptually, pain can be thought of as being composed of three hierarchical levels: a sensory-discriminative component (e.g., location, intensity, quality), a motivational-affective component (e.g., depression, anxiety), and a cognitive-evaluative component (e.g., thoughts concerning the cause and significance of the pain). The perception of pain is supported by a system of sensory neurons (nociceptors), and neural afferent pathways that specifically respond to potentially noxious, tissue-damaging stimuli. Activity in these pathways can be influenced by non-nociceptive pathophysiologic (e.g., abnormal nervous system processing) or psychological factors.

Pain may be a symptom of an underlying disease or disorder, or a condition in its own right. Pain can generally be divided into two types: acute and chronic. The distinction between acute and chronic pain is not based on its duration of sensation, but rather the nature of the pain itself. The primary distinction is that acute pain serves to protect one after an injury, while chronic pain does not serve this or any other purpose. Acute pain is the symptom of pain; chronic pain is the disease of pain.

Acute pain, for the most part, results from disease, inflammation, or injury to tissues. This type of pain generally comes on suddenly, for example, after trauma or surgery, and may be accompanied by anxiety or emotional distress. The cause of acute pain can usually be diagnosed and treated, and the pain is often self-limiting, i.e., it is confined to a given period of time and severity. In some instances, it can become chronic.

Chronic pain is widely believed to be a disorder itself. It can be made worse by environmental and psychological factors. Chronic pain persists over a longer period of time than acute pain and can be resistant to many medical treatments.

Based on clinical characteristics, inferences can be made about the predominating types of mechanisms sustaining pain. A classification based on inferred pathophysiology broadly divides pain syndromes into nociceptive, neuropathic, psychogenic, mixed, or idiopathic.

*Nociceptive Pain Mechanisms:* Clinically, pain can be labeled "nociceptive" if it can be inferred that the pain is related to nociceptor stimulation and subsequent activation of sensory nerve fibers by processes causing tissue injury. Nociceptive pain involves the normal activation of the nociceptive system by noxious stimuli. Nociception consists of four processes: transduction, transmission, perception, and modulation.

Normal somatosensory processing involves interaction between afferent systems activated by tissue injury and accompanying inflammation. The primary afferent system includes nociceptors (A-δ and C- fibers), signal processing in the dorsal horn of the spinal cord, ascending neural pathways, and thalamic and other specialized brain structures. Peripheral nociceptors are lightly myelinated (A-δ fibers) or non-myelinated (C fibers) ends of primary afferent nociceptive (sensory) neurons. Peripheral nociceptors have various response characteristics and they can be found in skin, muscle, joints, and some visceral tissues.

The nociceptive process begins with transduction (depolarization) at the peripheral nociceptors in response to noxious stimuli. Transmission is the process by which these stimuli proceed along primary afferent nociceptive axons to the spinal cord and then on to higher centers. Only when the impulses reach the brain are they intellectually recognized as pain. This is perception.

The ultimate perception of pain depends on both activity in this afferent system and its modulation at multiple levels of the nervous system. Pain modulation is determined by activity in the endogenous opioidergic system and other pain modulating systems. In the opioidergic system, analgesia is mediated by the binding of endogenous opioid compounds to opoiod receptors, mainly mu, delta, and kappa opoid receptors. Endogenous opioids are widely distributed and closely associated with systems known to regulate homeostasis, response to stress, and pain. In this complex system, other neurotransmitters, such as serotonin and norepinephrine, also play a role in the endogenous pain modulating system.

Nociceptive pain can be acute (short-lived, remitting) or persistent (long-lived, chronic), and may primarily involve injury to somatic or visceral tissues. Pain due to activation of somatic primary afferents is termed somatic pain and is typically localized and described as aching, squeezing, stabbing, or throbbing. Arthritis and metastatic bone pain are examples of somatic pain. Pain arising from stimulation of afferent receptors in the viscera is referred to as visceral pain. Visceral pain caused by obstruction of hollow viscus is poorly localized (because most viscera do not contain nociceptors) and is often described as cramping and gnawing, with a daily pattern of varying intensity. When organ capsules are involved, the pain may be described as sharp, stabbing or throbbing, descriptors similar to those associated with somatic pain.

Nociceptive pain may involve acute or chronic inflammation. The physiology of inflammation is complex. In addition to the activity of inflammatory mediators released by inflammatory and/or immune cells or generated by plasma components on the nociceptors, , retrograde release of substances from C polymodal nociceptors also may be involved. This "neurogenic inflammation" involves the release of the endogenous pain facilitatory chemical known as substance P, as well as serotonin, histamine, acetylcholine, and bradykinin. These substances activate and sensitize other nociceptors. Prostaglandins produced at the site of injury act to further enhance the nociceptive response to inflammation by lowering the threshold to noxious stimulation. Chronic inflammation with nociceptive stimulation may be the source of persistent pain.

*Examples of Pain:* There are many examples of pain and causes of pain. Examples include pain of childbirth, pain of a heart attack, pain that accompanies or follows surgery, pain that follows amputation of a limb, pain accompanying cancer, and pain that follows severe trauma, such as that associated with head and spinal cord injuries. Other, non limiting, examples and causes include:
Arachnoiditis: a condition in which one of the three membranes covering the brain and spinal cord, called the arachnoid membrane, becomes inflamed. A number of causes, including infection or trauma, can result in inflammation of this membrane. Arachnoiditis can produce disabling, progressive, and even permanent pain.

Arthritis, e.g., arthritic conditions, such as: osteoarthritis, rheumatoid arthritis, ankylosing spondylitis, and gout. These disorders are characterized by joint pain in the extremities.

Inflammatory diseases that affect the body's soft tissues, including tendonitis and bursitis.

Back pain: a common cause of disability for people, especially in the lower back. Back pain includes sciatica, which spreads to the leg. Another common type of back pain is associated with the discs of the spine. Discs protect the spine by absorbing shock, but they tend to degenerate over time and may sometimes rupture. Spondylolisthesis is a back condition that occurs when one vertebra extends over another, causing pressure on nerves and therefore pain. Damage to nerve roots (radiculopathy) is a serious condition and can be extremely painful.

Burn pain: e.g., associated with first, second, or third degree burns. Even after the wound has healed, subjects may have chronic pain at the burn site.

Cancer pain: this pain can accompany the growth of a tumor or metastasis of a cancer or tumor, the treatment of cancer, or chronic problems related to cancer's permanent effects on the body.

Headaches: including chronic headaches such as migraines, cluster headaches, and tension headaches. Migraines are characterized by throbbing pain and sometimes by other symptoms, such as nausea and visual disturbances. Stress can trigger a migraine headache, and migraines can also put the sufferer at risk for stroke. Cluster headaches are characterized by excruciating, piercing pain on one side of the head. Tension headaches are often described as a tight band around the head.

Head and facial pain can be agonizing, and can result, for example, from dental problems or from disorders such as cranial neuralgia, in which one of the nerves in the face, head, or neck is inflamed. Trigeminal neuralgia affects the largest of the cranial nerves and is characterized by a stabbing, shooting pain.

Muscle pain can range from an aching muscle, spasm, or strain, to the severe spasticity that accompanies paralysis. Another disabling syndrome is fibromyalgia, a disorder characterized by fatigue, stiffness, joint tenderness, and widespread muscle pain. Polymyositis, dermatomyositis, and inclusion body myositis are painful disorders characterized by muscle inflammation. They may be caused by infection or autoimmune dysfunction and are sometimes associated with connective tissue disorders, such as lupus and rheumatoid arthritis.

Myofascial pain syndromes affect sensitive areas known as trigger points located within the body's muscles. Fibromyalgia is a type of myofascial pain syndrome.

Neuropathic pain is a type of pain that can result from injury to nerves, either in the peripheral or central nervous system. Neuropathic pain can occur in any part of the body and is frequently described as a hot, burning sensation. It can result from diseases that affect nerves (such as diabetes) or from trauma, or it can be a consequence of cancer treatment, as chemotherapy drugs can affect nerves. Among the many neuropathic pain conditions are trigeminal neuralgia, diabetic neuropathy (which results from nerve damage secondary to vascular problems that occur with diabetes); reflex sympathetic dystrophy syndrome, which can follow injury; phantom limb and post-amputation pain, which can result from the surgical removal of a limb; postherpetic neuralgia, which can occur after an outbreak of shingles; and central pain syndrome, which can result from trauma to the brain or spinal cord.

Reflex sympathetic dystrophy syndrome, or RSDS: is accompanied by burning pain and hypersensitivity to temperature. RSDS is often triggered by trauma or nerve damage, RSDS causes the skin of the affected area to become characteristically shiny. In recent years, RSDS has come to be called complex regional pain syndrome (CRPS); in the past it was often called causalgia.

Repetitive stress injuries: muscular conditions that result from repeated motions performed in the course of normal work or other daily activities. They include: writer's cramp, which affects musicians and writers and others, compression or entrapment neuropathies, including carpal tunnel syndrome, caused by chronic overextension of the wrist and tendonitis or tenosynovitis, affecting one or more tendons.

Sciatica: a painful condition caused by pressure on the sciatic nerve, the main nerve that branches off the spinal cord and continues down into the thighs, legs, ankles, and feet. Sciatica is characterized by pain in the buttocks and can be caused by a number of factors. Exertion, obesity, and poor posture can all cause pressure on the sciatic nerve. One common cause of sciatica is a herniated disc.

Shingles and other painful disorders affect the skin: pain is a common symptom of many skin disorders, including rashes. Shingles (herpes zoster) is an infection that can cause agonizing pain resistant to treatment. Prompt treatment with antiviral agents is important to arrest the infection, which if prolonged can result in an associated condition known as postherpetic neuralgia. Other painful disorders affecting the skin include: vasculitis, or inflammation of blood vessels; other infections, including herpes simplex; skin tumors and cysts, and tumors associated with neurofibromatosis, a neurogenetic disorder.

Sports injuries: such as sprains, strains, bruises, dislocations, and fractures can cause pain. In extreme cases, sports injuries can take the form of costly and painful spinal cord and head injuries.

Spinal stenosis: a narrowing of the canal surrounding the spinal cord. The condition can occur naturally with aging. Spinal stenosis causes weakness in the legs and leg pain usually felt while the person is standing up and often relieved by sitting down.

Surgical pain: Surgery may require regional or general anesthesia during the procedure and/or medications to control discomfort following the procedure.

Temporomandibular disorders: conditions in which the temporomandibular joint (the jaw) is damaged and/or the muscles used for chewing and talking become stressed. The condition may be the result of a number of factors, such as an injury to the jaw or joint misalignment, and may give rise to a variety of symptoms, most commonly pain in the jaw, face, and/or neck muscles.

Trauma: which can occur, for example, after injuries in the home, at the workplace, during sports activities, or on the road, etc. Any of these injuries can result in severe disability and minor or severe pain. Some subjects who have had an injury to the spinal cord experience intense pain ranging from tingling to burning or both. Such subjects may be sensitive to hot and cold temperatures and touch. A touch can be perceived as intense burning, indicating abnormal signals relayed to and from the brain. This condition is called central pain syndrome or, if the damage is in the thalamus (the brain's center for processing bodily sensations), thalamic pain syndrome. It can affect subjects with multiple sclerosis, Parkinson's disease, amputated limbs, spinal cord injuries, or stroke.

Vascular disease or injury-such as vasculitis or inflammation of blood vessels, coronary artery disease, and circulatory problems: Vascular pain affects millions of Americans and occurs when communication between blood vessels and nerves is interrupted. Ruptures, spasms, constriction, or obstruction of blood vessels, as well as ischemia in which blood supply to organs, tissues, or limbs is cut off, can also result in pain. Pain associated with vascular disease also includes post cerebralvascular accidents (e.g., stroke).

Other examples of pain that can be treated by the compounds and methods prescribed herein include pain caused by: sympathetic dystrophy, neoplasia-associated pains, dental pain, dysmenorrhea, renal, menstrual or biliary colic, joint pains, intra-ocular hypertension, post-arthroscopy pain, post-laparoscopy gynecological pain, pain produced by percutaneous nephrolithotomy, radical retropubic post-prostatectomy pain, post-thoracotomy pain, post-tonsillectomy pain in pediatric subjects, post-hysterectomy pain, cesarean post-operation pains, cocaine or opioid dependence or withdrawal, cellular proliferation, small cell pulmonary carcinoma, depression and psychosis, inflammation, conditions due to increase of angiogenesis activity, wounds, coronary ischemic diseases, Parkinson's disease and dyskinesias, hepatic encephalopathy, cognitive diseases, Alzheimer's disease, itch, e.g., itch due to hepatic cholestasis or hyperinsulinemia in women with polycystic ovary.

### Sources of Pain

The experience of pain can be grouped according to the source and the involvement of nociceptors (if any).

Cutaneous pain is caused by injury to the skin or superficial tissues. Cutaneous nociceptors terminate just below the skin, and due to the high concentration of nerve endings, produce a well-defined, localized pain of short duration. Examples of injuries that produce cutaneous pain include paper cuts, minor cuts, minor (first degree) burns and lacerations.

Somatic pain originates from ligaments, tendons, bones, blood vessels, and even nerves themselves. It is detected with somatic nociceptors. The scarcity of pain receptors in these areas produces a dull, poorly-localized pain of longer duration than cutaneous pain; examples include sprains and broken bones.

Visceral pain originates from body's viscera, or organs. Visceral nociceptors are located within body organs and internal cavities. The even greater scarcity of nociceptors in these areas produces pain that is usually more aching and of a longer duration than somatic pain. Visceral pain is extremely difficult to localize, and several injuries to visceral tissue exhibit "referred" pain, where the sensation is localized to an area completely unrelated to the site of injury. Myocardial ischaemia (the loss of blood flow to a part of the heart muscle tissue) is possibly the best known example of referred pain; the sensation can occur in the upper chest as a restricted feeling, or as an ache in the left shoulder, arm or even hand. Referred pain can be explained by the findings that pain receptors in the viscera also excite spinal cord neurons that are excited by cutaneous tissue. Since the brain normally associates firing of these spinal cord neurons with stimulation of somatic tissues in skin or muscle, pain signals arising from the viscera are interpreted by the brain as originating from the skin.

Phantom limb pain is the sensation of pain from a limb that has been lost or from which a person no longer receives physical signals.

Neuropathic pain (also known as neuralgia) is the label applied to pain syndromes inferred to result from direct injury or dysfunction of the sensory axons in the peripheral or central nervous system (CNS). These changes may be caused by injury to either neural or non-neural tissues. Although neuropathic pain is influenced by ongoing tissue injury, there is an assumption that the fundamental mechanisms sustaining the pain have become independent of the initial injury or damage. The pain may or may not be lacinating. Neuropathic pain syndromes may be associated with referred pain, allodynia (pain induced by non-noxious stimuli, e.g. light touch), hyperalgesia (increased response to a noxious stimuli), or hyperpathia (exaggerated responses to painful stimuli, with continuing sensation of pain after the stimulation has ceased).

Some of the neurophysiologic and neuroanatomic changes that may occur in peripherally-generated neuropathic pain are understood. Injury to peripheral neural axons can result in abnormal nerve regeneration in the weeks to months following injury. The damaged axon may grow multiple nerve sprouts, some of which form neuromas. These nerve sprouts, including those forming neuromas, can generate spontaneous activity, which peaks in intensity several weeks after injury. Unlike normal axons, these structures are more sensitive to physical distention, which is clinically associated with tenderness and the appearance of Tinel's sign (i.e., sensation of tingling or "pins and needles" when the area is tapped or manipulated). After a period of time, atypical connections may develop between nerve sprouts or demyelinated axons in the region of the nerve damage, permitting "cross-talk" between somatic or sympathetic efferent nerves and nociceptors. Dorsal root fibers may also sprout following injury to peripheral nerves.

### Nociceptors

Nociceptors are free nerve endings that have their cell bodies outside the spinal column in the dorsal root ganglion. There are mechanical, thermal, and chemical nociceptors. They are found in skin and on internal surfaces such as periosteum and joint surfaces. Deep internal surfaces are only weakly supplied with pain receptors and will propagate sensations of chronic, aching pain if tissue damage in these areas occurs.

Two main types of nociceptor, Aδ and C nociceptors, mediate fast and slow pain respectively. Thinly myelinated type Aδ fibers mediate fast pain that can be described as sharp, acute, pricking pain and includes mechanical and thermal pain (mediated by Aδ mechanosensitive nociceptors and Aδ mechanothermal nociceptors respectively). Slow pain, mediated by slower, unmyelinated type C pain fibers, is an aching, throbbing, burning pain. Polymodal nociceptors, which respond to thermal, mechanical, and chemical stimuli, are a type of C nociceptor. Chemical pain is an example of slow pain. Nociceptors do not adapt to stimulus. In some conditions, excitation of pain fibers becomes greater as the pain stimulus continues, leading to hyperalgesia.

Transmission of nociception signals in the central nervous system: There are two pathways for transmission of nociception in the central nervous system: the neospinothalamic tract (for fast pain) and the paleospinothalamic tract (for slow pain).

Fast pain travels via type Aδ fibers to terminate on lamina I (lamina marginalis) of the dorsal horn of the spinal cord. Second order neurons of the neospinothalamic tract then take off and give rise to long fibers which cross the midline through the grey commisure and pass upwards in the contralateral anterolateral columns. These fibers then terminate on the reticular formation, Ventrobasal Complex (VBC) of the thalamus. From here, third order neurons communicate with the somatosensory cortex.

Slow pain is transmitted via slower type C fibers to laminae II and III of the dorsa horns, together known as the substantia gelatinosa. Second order neurons take off and terminate in lamina V, also in the dorsal horn. Third order neurons then join fibers from the fast pathway, crossing to the opposite side via the grey commisure, and traveling upwards through the anterolateral pathway. These neurons terminate widely in the brain stem, with one tenth of fibers stopping in the thalamus, and the rest stopping in the medulla, pons and tectum of midbrainmesencephalon, periaqueductal grey.

Consequences of nociception: When the nociceptors are stimulated, they transmit signals through sensory neurons in the spinal cord. These neurons release glutamate, a major exicitory neurotransmitter that relays signals from one neuron to another.

If the signals are sent to the reticular formation of brain stem, thalamus, then pain enters consciousness, but in a dull poorly localized manner. From the thalamus, the signal can travel to the somatosensory cortex in the cerebrum, when the pain is experienced as localized and having more specific qualities.

### Pain Diagnosis

There is no completely accurate method to measure pain. Sometimes, as in the case of headaches, the best aid to diagnosis is the subject's own description of the type, duration, and location of pain.

A number of technologies can be employed to identify the cause of pain. Examples include:
Electrodiagnostic procedures include electromyography (EMG), nerve conduction studies, and evoked potential (EP) studies. Information from EMG can identify precisely which muscles or nerves are affected by weakness or pain. Thin needles are inserted in muscles and a physician can see or listen to electrical signals displayed on an EMG machine. With nerve conduction studies, two sets of electrodes (similar to those used during an electrocardiogram) are placed on the skin over the muscles. The first set gives the subject a mild shock that stimulates the nerve that runs to that muscle. The second set of electrodes is used to make a recording of the nerve's electrical signals, and from this information the presence of nerve damage can be determined. EP tests also involve two sets of electrodes-one set for stimulating a nerve (these electrodes are attached to a limb) and another set on the scalp for recording the speed of nerve signal transmission to the brain.

Imaging, especially magnetic resonance imaging (MRI), provides images of the body's structures and tissues. MRI uses magnetic fields and radio waves to differentiate between healthy and diseased tissue.

A neurological examination in which movement, reflexes, sensation, balance, and coordination are tested.

X-rays may also be used, e.g., to evaluate bones and joints.

### Analgesia

The endogenous analgesic system is mediated by three major components: the periaquaductal grey matter (in the midbrain), the nucleus raphe magnus (in the medulla), and the nociception inhibitory neurons within the dorsal horns of the spinal cord, which act to inhibit nociception-transmitting neurons also located in the spinal dorsal horn.

Anti-nociceptive effects can be mediated through opioid receptors. Several different types of opioid receptors are activated in response to the binding of endogenous opioid peptides. These receptors, which exist in a variety of areas in the body, inhibit firing of neurons that would otherwise be stimulated to do so by nociceptor activation. Thus, activation of the opioid receptors (e.g., with a peptide described herein) can cause an anti-nociceptive and analgesic effect.

Opioid receptors are a group of G-protein coupled receptors with opioids as ligands. The endogenous opioids are dynorphins, enkephalins and endorphins. There are three major subtypes of opioid receptors: µ (mu), κ (kappa), and δ (delta). An orphan opioid receptor (ORLI) has been identified. Several receptor subtypes (µ1, µ2; κ1, κ2, κ3; δ1, δ2) have been identified in human tissue.

*Mu receptor:* The µ opioid receptors can exist either presynaptically or postsynaptically depending upon cell types. The µ receptors exist mostly presynaptically in the periaqueductal gray region, and in the superficial dorsal horn of the spinal cord. Other areas where µ-receptors have been located include the external plexiform layer of the olfactory bulb, the nucleus accumbens, in several layers of the cerebral cortex and in some of the nuclei of the amygdala. Data have also indicated the presence of µ opioid receptors in the periphery, including inflammatory cells and peripheral nerve endings The µ receptor has high affinity for enkephalins and beta-endorphin but low affinity for dynorphins. The opioid alkaloids morphine and codeine bind to the mu receptor. Activation of the µ receptor by an agonist such as morphine causes analgesia, sedation, reduced blood pressure, itching, nausea, euphoria, decreased respiration, miosis (constricted pupils) and decreased bowel motility often leading to constipation. Some of these effects, such as sedation, euphoria and decreased respiration, tend to disappear with continued use as tolerance develops. Tolerance to the analgesic effect is also observed. Miosis and reduced bowel motility tend to persist; little tolerance develops to these effects. Tolerance develops to different effects at different rates largely because these effects are caused by activation of different µ-receptor subtypes. Stimulation of µ1-receptors blocks pain while stimulation of µ2-receptor causes respiratory depression and constipation.

*Kappa receptor:* κ Opioid receptors are also involved with analgesia, but activation also produces marked nausea and dysphoria. Kappa ligands are also known for their characteristic diuretic effects, due to their negative regulation of anti diuretic hormone (ADH). Kappa agonism is neuroprotective against hypoxia/ischemia, as such, kappa receptors may represent a novel therapeutic target. The endogenous ligands for the kappa receptor are the dynorphins. Kappa receptors are located in the periphery, in sensory neurons, in the spinal cord and in the brain. Kappa agonists can produce psychotomimetic effects. A drug known as ketocyclazocine binds to kappa receptors.

*Delta receptor:* δ Opioid receptor activation also produces analgesia. Some research suggests that they may also be related to seizures. The endogenous ligands for the δ receptor are the enkephalins. Exogenous ligands which activate the delta receptors can mimic the phenomenon known as "ischemic preconditioning." Experimentally, if short periods of transient ischemia are induced, downstream tissues are robustly protected if permanent interruption of the blood supply is then effected. Opiates and opioids with delta activity mimic this effect. In the rat model, introduction of delta active ligands results in significant cardioprotection.

Analgesics (e.g., the peptides described herein) can cause an analgesic effect by acting on a nociceptive mechanism of pain. For example, the peptides described herein can induce an analgesic effect mediated by the activation of opioid receptors, particularly the kappa and/or delta opioid receptors, and cause an anti-nociceptive effect (and analgesic effect).

### Pain Treatment

The goal of pain treatment (e.g., management) is to improve function, enabling subjects to work, attend school, participate in other day-to-day activities, relieve suffering, and/or improve the quality of life for the pain sufferer. Numerous treatments are available. Examples include:
Peptides (e.g., analgesic and anti-nociceptive peptides) described herein. One or a combination of peptides (e.g., more than one of the peptides described herein) can be used to treat pain.

Acetaminophen which can be sold over the counter, in a prescription-strength preparation, or can be formulated in combination with codeine.

Acupuncture which involves the application of needles to precise points on the body.

Analgesics which are a class of drugs that includes most painkillers, such as aspirin, paracetamol (acetaminophen), and ibuprofen, e.g., and additional agents listed herein. Other examples include the nonsteroidal anti-inflammatory drugs (NSAIDs) such as salicylates, COX2 inhibitors, narcotic drugs such as morphine, synthetic drugs with narcotic properties such as tramadol, Acetaminophen (Tylenol), Codeine (Tylenol #2,3,4), Darvocet (Propoxyphene/Acetaminophen), Darvon (Propoxyphene), Duragesic (Fentanyl Patch), Hydromorphone (Palladone, Dilaudid,) Morphine (MSContin, Oramorph), Oxycodone (OxyContin, Roxicodone), Percocet (Oxycodone/Acetaminophen), Percodan (Oxycodone/Aspirin), Talwin NX (Pentazocine/Naloxone), Ultracet (Tramadol/Acetaminophen), Ultram (Tramadol), and Vicodin (Hydrocodone/Acetaminophen).

Anticonvulsants are used for the treatment of seizure disorders and may also be sometimes prescribed for the treatment of pain. Carbamazepine in particular is used to treat a number of painful conditions, including trigeminal neuralgia. Another antiepileptic drug, gabapentin, is being studied for its pain-relieving properties, especially as a treatment for neuropathic pain.

Antidepressants are sometimes used for the treatment of pain and, along with neuroleptics and lithium, are classified as psychotropic drugs. In addition, anti-anxiety drugs called benzodiazepines also act as muscle relaxants and are sometimes used as pain relievers.

Antimigraine drugs include the triptans- sumatriptan (IMITREX®), naratriptan (AMERGE®), and zolmitriptan (ZOMIG®)-and are used specifically for migraine headaches.

Aspirin may be the most widely used pain-relief agent and has been sold over the counter since 1905 as a treatment for, inter alia, fever, headache, and muscle soreness.

Biofeedback is used for the treatment of many common pain problems, most notably headache and back pain. Using a special electronic machine, the subject is trained to become aware of, to follow, and to gain control over certain bodily functions, including muscle tension, heart rate, and skin temperature. The subject can then learn to effect a change in his or her responses to pain, for example, by using relaxation techniques. Similarly, the use of relaxation techniques in the treatment of pain can increase the subject's feeling of well-being.

Capsaicin is a chemical found in chili peppers that is also a primary ingredient in pain-relieving creams.

Chemonucleolysis is a treatment in which an enzyme, chymopapain, is injected directly into a herniated lumbar disc in an effort to dissolve material around the disc, thus reducing pressure and pain.

Chiropractic refers to hand manipulation of the spine, usually for relief of back pain.

Cognitive-behavioral therapy involves a wide variety of coping skills and relaxation methods to help prepare for and cope with pain. It is used for postoperative pain, cancer pain, and the pain of childbirth.

Counseling can give a subject suffering from pain much needed support. Support groups can provide an important adjunct to drug or surgical treatment. Psychological treatment can also help subjects learn about the physiological changes produced by pain.

COX-2 inhibitors may be effective, e.g., for subjects with arthritis. COX-2 inhibitors (e.g., celecoxib) primarily block cyclooxygenase-2 and are less likely to have the gastrointestinal side effects sometimes produced by NSAIDs.

Electrical stimulation, including transcutaneous electrical stimulation (TENS), implanted electric nerve stimulation, and deep brain or spinal cord stimulation, is a practice in which the nerves of muscles are subjected to a variety of stimuli, including heat or massage:
- TENS uses tiny electrical pulses, delivered through the skin to nerve fibers, to cause changes in muscles, such as numbness or contractions. This in turn produces temporary pain relief. There is also evidence that TENS can activate subsets of peripheral nerve fibers that can block pain transmission at the spinal cord level.
- Peripheral nerve stimulation uses electrodes placed surgically on a carefully selected area of the body. The subject is then able to deliver an electrical current as needed to the affected area, using an antenna and transmitter.
- Spinal cord stimulation uses electrodes surgically inserted within the epidural space of the spinal cord. The subject is able to deliver a pulse of electricity to the spinal cord using a small box-like receiver and an antenna taped to the skin.
- Deep brain or intracerebral stimulation is considered an extreme treatment and involves surgical stimulation of the brain, usually the thalamus. It is used for a limited number of conditions, including severe pain, central pain syndrome, cancer pain, phantom limb pain, and other neuropathic pains.

Exercise can be a common treatment for pain. Because there is a known link between many types of chronic pain and tense, weak muscles, exercise can contribute to an overall sense of well-being by improving blood and oxygen flow to muscles. As stress can contribute to pain, exercise, sleep, and relaxation can help reduce stress, thereby helping to alleviate pain. Exercise has been proven to help many people with low back pain.

Hypnosis can used to control physical function or response, that is, the amount of pain a subject can withstand. Hypnosis may result in relief of pain by acting on chemicals in the nervous system, slowing impulses.

Ibuprofen is a member of the aspirin family of analgesics, the so-called nonsteroidal anti-inflammatory drugs.

Low-power lasers have been used occasionally by some physical therapists as a treatment for pain.

Magnets can be used, e.g., to treat sports-related pain and other painful conditions. The magnet is usually worn as a collar or wristwatch. Magnets may effect changes in cells or body chemistry, thus producing pain relief.

Muscle relaxants are a group of drugs that each have an overall sedative effect on the body. These drugs do not act directly on the muscles, rather they act centrally and are more of a total body relaxant. Typically, muscle relaxants are prescribed to relieve pain associated with muscle spasms. There are several types of muscle relaxant medications that are commonly used: carisoprodol (Soma), cyclobenzaprine (Flexeril), and diazepam (Valium).

Nerve blocks employ the use of drugs, chemical agents, or surgical techniques to interrupt the relay of pain messages between specific areas of the body and the brain. Types of surgical nerve blocks include neurectomy; spinal dorsal, cranial, and trigeminal rhizotomy; and sympathectomy, also called sympathetic blockade.

Nonsteroidal anti-inflammatory drugs (NSAIDs) (including aspirin and ibuprofen) are widely prescribed and sometimes called non-narcotic or non-opioid analgesics. Nonsteroidal anti-inflammatory drugs (NSAIDs) work by blocking two enzymes, cyclooxygenase-1 and cyclooxygenase-2, both of which promote production of hormones called prostaglandins, which in turn cause inflammation, fever, and pain.

Narcotics are effective pain treatments and include opioids. They include codeine and morphine. Morphine can be administered in a variety of forms, including a pump for subject self-administration. Opioids have a narcotic effect, that is, they induce sedation as well as pain relief, and some subjects may become physically dependent upon them. Other examples of opioids include: Alfentanil, Allylprodine, Alphaprodine, Anileridine, Betmeprodine, Betaprodine, Bezitramide, Buprenorphine, Butorphanol, Carfentanil, Dextropropoxyphene, Dextromoramide, Dihydrocodeine, Dipipanone, Etorphine, Fentanyl, Diamorphine (Heroin), Hydromorphone, Hydrocodone, Levorphanol, Methadone, Metopon, Nalbuphine, Nicomorphine, Omnopon, Opium, Oxycodone, Oxymorphone, Pantopon, Papaveretum, Paregoric, Pentazocine, Pethidine(Meperidine), Phenoperidine, Piritramide, Prodine, Proheptazine, Propiram, Propoxyphene, Racemorphan, Remifentanil, Sufentanil, Tetrapon, Tilidine, Tramadol, and Trimeperidine.

Physical therapy and rehabilitation include using physical techniques and methods, such as heat, cold, exercise, massage, and manipulation, in the treatment of certain conditions. These may be applied to increase function, control pain, and speed the subject toward full recovery.

Pyrazolone is a class of nonsteroidal anti inflammatory drugs that have been used in the treatment of arthritis and other musculoskeletal and joint disorders. It contains a five-membered-ring lactam that is a derivative of pyrazole that has an additional keto (=O) group. Examples of derivatives include: Ampyrone, Metamizole, Phenazone, and Phenylbutazone.

R.I.C.E.-Rest, Ice, Compression, and Elevation-are four components prescribed by many orthopedists, coaches, trainers, nurses, and other professionals for temporary muscle or joint conditions, such as sprains or strains.

Steroids can be given topically, orally, or by injection. When injected, they can be given into a vein or muscle, directly into a joint or bursa, or around tendons and other soft tissue areas. For example, cortisone injections can be administered (e.g., epidurally) into the spine to treat back pain and/or to treat inflammation. Examples of orally administered steroids include prednisone and methylprednisolone.

Surgery may be required to relieve pain, especially pain caused by back problems or serious musculoskeletal injuries. Surgery may take the form of a nerve block or it may involve an operation to relieve pain from a ruptured disc. Surgical procedures for back problems include discectomy or, when microsurgical techniques are used, microdiscectomy, in which the entire disc is removed; laminectomy, a procedure in which a surgeon removes only a disc fragment, gaining access by entering through the arched portion of a vertebra; and spinal fusion, a procedure where the entire disc is removed and replaced with a bone graft. In a spinal fusion, the two vertebrae are then fused together. Other operations for pain include rhizotomy, in which a nerve close to the spinal cord is cut, and cordotomy, where bundles of nerves within the spinal cord are severed. Cordotomy is generally used only for the pain of terminal cancer that does not respond to other therapies. Another operation for pain is the dorsal root entry zone operation, or DREZ, in which spinal neurons corresponding to the subject's pain are destroyed surgically. Occasionally, surgery is carried out with electrodes that selectively damage neurons in a targeted area of the brain.

Physical external supports, such as splints, casts, braces, pillows, and mattresses can be used to relieve pain.

The treatments described herein can be used alone or in combination with one or more other treatments described herein. Treatments used in combination can be administered concomitantly or sequentially. Combination treatments can include treating a subject with two or more of the peptides described herein.

### Analgesic Peptides

The present disclosure provides for peptides that have analgesic properties that can be used for the treatment of pain, such as acute or chronic pain. The peptides can have an anti-nociceptive effect. The peptides can act as agonists or partial agonists of opioid receptors. In some embodiments, the peptides can act on opioid receptors (e.g., kappa-and/or delta-type opioid receptors), directly or indirectly. For example, a peptide described herein can directly activate an opioid receptor or it can induce the release of an endogenous opioid (e.g., and thereby indirectly affect the opioid receptor).

The application describes peptides having the sequence:
R1-R2-Ser-R4-R5-R6-R7-R8-Gly-R10-Ser-R12-Pro-R14 (SEQ ID NO: 1)
wherein:
R1 is pyroglutamate,
R2 is Phe or Trp or Tyr or Leu or Thr,
R4 is Pro or Arg,
R5 is Glx or Asx or Gly,
R6 is Asn or Gin or Leu,
R7 is any amino acid,
R8 is any amino acid except Lys,
R10 is Glx or Asx,
R12 is Glx or Lys, and
R14 is any amino acid,
with the proviso that if R7 is Cys, R14 is not Cys and if R14 is Cys, R7 is not Cys.

In preferred embodiments, such peptides possess an activity of the 7A14A peptide described herein, e.g., an analgesic effect. Exemplary peptides include peptides that contain, consist essentially of, or consist of a peptide of SEQ ID NO: 1 In preferred embodiments, R8 is Glx.

A particularly preferred peptide contains, consists essentially of, or consists of the amino acid sequence pGlu-Phe-Ser-Pro-Glu-Asn-Ala-Gln-Gly-Glu-Ser-Gln-Pro-AIa (SEQ ID NO:2).

Also described herein are peptides that include an amino acid sequence at least about 70%, at least about 75%, at least about 80%, at least about 82%, at least about 85%, at least about 86%, at least about 87%, at least about 88%, at least about 89%, at least about 90%, at least about 91 %, at least about 92%, at least about 93%, at least about 94%, at least about 95%, at least about 96%, at least about 97%, at least about 98%, at least about 99% identical or completely identical to a peptide containing, consisting essentially of, or consisting of SEQ ID NO:1 and peptides encoded by a nucleic acid that hybridizes under high stringency conditions to a nucleic acid encoding peptide of SEQ ID NO: 1. Such peptides can possess an activity of the 7A14A peptide described herein, e.g., an analgesic effect.

Also described herein are peptides that include an amino acid sequence at least about 70%, at least about 75%, at least about 80%, at least about 82%, at least about 85%, at least about 86%, at least about 87%, at least about 88%, at least about 89%, at least about 90%, at least about 91 %, at least about 92%, at least about 93%, at least about 94%, at least about 95%, at least about 96%, at least about 97%, at least about 98%, at least about 99% identical or completely identical to a peptide containing, consisting essentially of, or consisting of SEQ ID NO:2 and peptides encoded by a nucleic acid that hybridizes under high stringency conditions to a nucleic acid encoding peptide of SEQ ID NO:2. Such peptides can possess an activity of the 7A14A peptide described herein, e.g., an analgesic effect.

Calculations of "homology" or "sequence identity" between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). The optimal alignment is determined as the best score using the GAP program in the GCG software package with a Blossum 62 scoring matrix with a gap penalty of 12, a gap extend penalty of 4, and a frameshift gap penalty of 5. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences. Alignments of related proteins described herein are instructive for identifying amino acid positions that tolerate modification, e.g., insertion, deletion, and substitution, e.g., conservative or non-conservative substitution.

Aligning a first sequence with, for example, the second sequence 7A14A (pGlu-Phe-Ser-Pro-Glu-Asn-Ala-Gin-Gly-Glu-Ser-Gln-Pro-Ala (SEQ ID NO:2)) can permit reference of amino acids from the first sequence that correspond with amino acids of 7A14A, for example, the amino acid from the first sequence that corresponds with pGlu of 7A14A is denominated R1, the amino acid from the first sequence that corresponds with Phe is denominated R2, the amino acid that corresponds with the first Ser is R3, the amino acid that corresponds with first Pro is R4, the amino acid that corresponds with the first Glu is R5, the amino acid that corresponds with Asn is R6, the amino acid that corresponds with the first Ala is R7, the amino acid that corresponds with the first Gln is R8, the amino acid that corresponds with Gly is R9, the amino acid that corresponds with the second Glu is R10, the amino acid that corresponds with the second Ser is R11, the amino acid that corresponds with the second Gln is R12, the amino acid that corresponds with the second Pro is R13, and the amino acid that corresponds with the second Ala is R14.

As used herein, the term "hybridizes under high stringency conditions" describes conditions for hybridization and washing. Guidance for performing hybridization reactions can be found in Current Protocols in Molecular Biology, John Wiley & Sons, N.Y. (1989), 6.3.1-6.3.6. Aqueous and nonaqueous methods are described in that reference and either can be used. High stringency hybridization conditions include hybridization in 6X SSC at about 45°C, followed by one or more washes in 0.2X SSC, 0.1 % SDS at 65°C, or substantially similar conditions.

Also described herein are peptides that contain, consist essentially of, or consist of an amino acid sequence that differs from the sequence of SEQ ID NO:1 by one, two, three, four, five, six, seven, eight, nine, ten, eleven, twelve, thirteen, or fourteen amino acid substitutions, additions, or deletions. They can be at any position, e.g., internal or terminal (e.g., at the amino or carboxy terminus). Such peptides can possess an activity of the ENPAK or 7A14A peptide described herein, e.g., an analgesic effect.

Also described herein are peptides that contain, consist essentially of, or consist of an amino acid sequence that differs from the sequence of SEQ ID NO:1 by at least one, but not more than two, three, four, five, six, seven, eight, nine, ten, eleven, twelve, or thirteen, amino acid substitutions, additions, or deletions. They can be at any position, e.g., internal or terminal (e.g., at the N or C terminus). Such peptides can possess an activity of the ENPAK or 7A14A peptide described herein, e.g., an analgesic effect.

The application also describes peptides contain, consist essentially of, or consist of an amino acid sequence that differs from the sequence of SEQ ID NO:1 by one, two, three, four, five, six, seven, eight, nine, ten, eleven, twelve, thirteen, or fourteen conservative amino acid substitutions. They can be at any position, e.g., internal or terminal (e.g., at the N or C terminus). Such peptides can possess an activity of the ENPAK or 7A14A peptide described herein, e.g., an analgesic effect.

The application also describes peptides that contain, consist essentially of, or consist of an amino acid sequence that differs from the sequence of SEQ ID NO:2 by one, two, three, four, five, six, seven, eight, nine, ten, eleven, twelve, thirteen, or fourteen amino acid substitutions, additions, or deletions. They can be at any position, e.g., internal or terminal (e.g., at the N or C terminus). Such peptides can possess an activity of the ENPAK or 7A14A peptide described herein, e.g., an analgesic effect.

Also described are peptides that contain, consist essentially of, or consist of an amino acid sequence that differs from the sequence of SEQ ID NO:2 by at least one, but not more than two, three, four, five, six, seven, eight, nine, ten, eleven, twelve, or thirteen, amino acid substitutions, additions, or deletions. They can be at any position, e.g., internal or terminal (e.g., at the N or e terminus). Such peptides can possess an activity of the ENPAK or 7A14A peptide described herein, e.g., an analgesic effect.

The application also describes peptides contain, consist essentially of, or consist of an amino acid sequence that differs from the sequence of SEQ ID NO:2 by one, two, three, four, five, six, seven, eight, nine, ten, eleven, twelve, thirteen, or fourteen conservative amino acid substitutions. They can be at any position, e.g., internal or terminal (e.g., at the N or C terminus). Such peptides can possess an activity of the ENPAK or 7A14A peptide described herein, e.g., an analgesic effect.

Also described herein are fragments of ENPAK or 7A14A peptides, e.g., fragments that possess an activity of ENPAK or 7A14A peptide, e.g., an analgesic effect. The fragments can contain, e.g., the amino-terminal 5, 6, 7, or 8 amino acids of ENPAK or 7A14A. The fragments can contain, e.g., the carboxy-terminal 5, 6, 7, or 8 amino acids of ENPAK or 7A14A. The fragments can contain, e.g., amino acids of ENPAK or 7A14A that do not include a terminal peptide (e.g., do not contain the amino-or the carboxy-terminal residues) of the peptide, e.g., the fragments can contain 5, 6, 7, or 8 internal amino acids of ENPAK or 7A14A.

As used herein, the term "conservative amino acid substitution" describes the substitution of one amino acid for another with similar characteristics, e.g. substitutions within the following groups: valine, glycine; glycine, alanine; valine, isoleucine, leucine; aspartic acid, glutamic acid; asparagine, glutamine; serine, threonine; lysine, arginine; and phenylalanine, tyrosine. The following substitutions are further non-limiting examples of conservative subsitutions:
Alanine: replace with: D-Ala, Gly, Beta-Ala, L-Cys, D-Cys;
Arginine: replace with: D-Arg, Lys, D-Lys, homo-Arg, D-homo-Arg, Met,Ile, D-Met, D-Ile, Om, D-Om;
Asparagine: replace with: D-Asn, Asp, D-Asp, Glu, D-Glu, Gln, D-Gln;
Aspartic Acid: replace with: D-Asp, D-Asn, Asn, Glu, D-Glu, Gln, D-Gln;
Cysteine: replace with: D-Cys, S-Me-Cys, Met, D-Met, Thr, D-Thr;
Glutamine: replace with: D-Gln, Asn, D-Asn, Glu, D-Glu, Asp, D-Asp;
Glutamic Acid: replace with: D-Glu, D-Asp, Asp, Asn, D-Asn, GIn, D-Gln;
Glycine: replace with: Ala, D-Ala, Pro, D-Pro, Acp;
Isoleucine: replace with: D-Ile, Val, D-Val, Leu, D-Leu, Met, D-Met;
Leucine: replace with: D-Leu, Val, D-Val, Leu, D-Leu, Met, D-Met;
Lysine: replace with: D-Lys, Arg, D-Arg, Homo-arg, D-homo-Arg, Met, D-Met, Ile, D-ILe, Om, D-Om;
Methionine: replace with: D-Met, S-Me-Cys, Ile, D-Ile, Leu, D-Leu, Val, D-Val;
Phenylalanine: replace with: D-Phe, Tyr, D-Thr, L- Dopa, His, D-His, Trp, D-Trp, Trans-3, 4 or 5- phenylproline, cis-3, 4, or 5-phenylproline;
Proline: replace with: D-Pro, L-I-thoazolidine-4- carboxylic acid, D-or L-1-oxazolidine-4-carboxylic acid;
Serine: replace with: D-Ser, Thr, D-Thr, allo- Thr, Met, D-Met, Met(O), D-Met(O), L-Cys, D-Cys;
Threonine: replace with: D-Thr, Ser, D-Ser, allo- Thr, Met, D-Met, Met(O), D-Met(O), Val, D-Val;
Tyrosine: replace with: D-Tyr, Phe, D-Phe, L- Dopa, His, D-His;
Valine: replace with: D-Val, Leu, D-Leu, Ile, D- Ile, Met, D-Met.

The suitability of a variant (e.g., that carries one or more of amino acid substitutions, additions, or deletions) of a peptide sequence provided herein for use as an analgesic can be tested using a technique described herein. For example, one can test a candidate peptide (e.g., a peptide that differs from the peptide of SEQ ID NO:2 by an amino acid deletion, substitution, or addition) for its analgesic effects. The analgesic effect of the candidate peptide can be measured, e.g., in a prostaglandin E2-induced hyperalgesia model. The effects of the candidate peptide can be compared with one or more standards. For example, a suitable standard would be a peptide described herein, e.g., a peptide consisting of the amino acid sequence of SEQ ID NO:2. The analgesic effects of the candidate peptide and the peptide of SEQ ID NO:2, wherein both peptides are used at the same concentration and are administered by the same route of administration, are compared. Suitability can be shown by the candidate causing a better or as good of an analgesic effect as the standard. Alternatively or in addition, suitability can be shown by the candidate causing an analgesic effect that lasts longer than or as long as the analgesic effect caused by the standard.

One or more of the amino acids of SEQ ID NO:1 and SEQ ID NO:2 and SEQ ID NO:3 can be D- or L- amino acids. The amino acids can be naturally occurring or non-naturally occurring (e.g., synthetic) analogs. The amino acids can be modified. Modifications include: acetylation (e.g., at the N-terminus of the peptide); alkylation (e.g., methylation); biotinylation, acylation; biotinylation; glutamylation; glycosylation (e.g., N- or O- glycosylation); isoprenylation (e.g., addition of farnesol and geranylgeraniol); lipoylation; phosphopantetheinylation; phosphorylation; sulfation; selenation; amidation (e.g., C-terminal amidation); ubiquitination; citrullination/deimination; deamidation;; proteolytic cleavage; formylation; myristoylation; pyroglutamate addition; carbamylation; glycosyl phosphatidylinositol (GPI) addition; O-methylation; glypiation; sumoylation; acylation; hydroxylation; desmosine addition; deamination; oxidation (e.g., to aldehyde); imine formation; glycation; carbamylation; disulfide bond formation (e.g., creation of intra- or inter- molecular disulfide bridges); prenylation; palmitoylation; porphyrin ring linkage; flavin linkage; GFP prosthetic group (Thr-Tyr-Gly sequence) formation; lysine tyrosine quinone (LTQ) formation; topaquinone (TPQ) formation; succinimide formation; transglutamination; carboxylation; polyglutamylation; polyglycylation.

The N- and/or C- terminus of the peptide can be modified to increase stability, e.g., to decrease degradation, e.g., proteolytic degradation.

The peptides can be modified to contain an epitope tag (e.g., a His (e.g., 6x His or poly-His), Myc, HA, GST, MBP, VSV, Thioredoxin, Beta-Gal, FLAG, or GFP tag, etc.) for example, to aid in the identification or purification of the peptide. Such a tag can be present, for example, on the N- or C- terminus of the peptide. A cleavage site (e.g., a recognition site for Factor Xa Protease, enterokinase, thrombin, TEV protease, PRESCISSION™ protease, intein 1 or intein 2, or a signal peptidase, etc.) can optionally be situated between the tag and peptide sequence so that the tag can be cleaved from the peptide. Such techniques are known in the art. See also Current Protocols in Molecular Biology 3rd ed., John Wiley and Sons, Inc, New York, NY.

### Peptide Preparation

The peptides described herein can be prepared in a biological system or chemically synthesized.

To produce the peptides in a biological system, the peptide can be produced by recombinant DNA technology. For example, an expression vector containing a nucleic acid sequence encoding a peptide described herein can be introduced into a biological system (e.g., bacterial, yeast, plant, insect, or mammalian expression system) and expressed using standard techniques. The peptide is then purified from the biological system (e.g., from cells or culture medium) using standard purification techniques (e.g., using separation techniques based on the physical or chemical properties of the peptide or affinity purification techniques). Such techniques are known in the art. See, e.g., Current Protocols in Molecular Biology 3rd ed., John Wiley and Sons, Inc, New York, NY.

The peptides can be chemically synthesized, e.g., using liquid or solid phase synthesis. Such techniques are standard in the art, see, e.g., Atherton, E., Sheppard, R.C. Solid Phase peptide synthesis: A practical approach. IRL Press, Oxford, England, 1989; Stewart J.M., Young, J.D. Solid phase peptide synthesis, 2nd edition, Pierce Chemical Company, Rockford, 1984; Carpino, L. A. 1992 1-Hydroxy-7-azabenzotriazole. An efficient Peptide Coupling Additive. J. Am. Chem. Soc. 115, 4397-4398. Peptides are synthesized by coupling the carboxyl group or C-terminus of one amino acid to the amino group or N-terminus of another.

Liquid-phase synthesis: Liquid-phase peptide synthesis is a classical approach to peptide synthesis. It is useful in large-scale production of peptides for industrial purposes.

Solid-phase synthesis: Solid-phase peptide synthesis (SPPS) is now the widely accepted method for creating peptides and proteins in the lab in a synthetic manner. SPPS allows, for example, the synthesis of natural peptides which are difficult to express in bacteria, the incorporation of unnatural amino acids, peptide/protein backbone modification, and the synthesis of D-proteins and peptides, contain D-amino acids. The synthesis can be performed manually, e.g., as described herein, or by employing an automated synthesizer.

Small beads are treated with linkers on which peptide chains can be built. The synthesis beads will retain strong bondage to the peptides until cleaved by a reagent, such as trifluoroacetic acid. The beads create a synthesis environment in which the peptide chains being created will not pass through a filter material while the reagents used to create them will.

There are two commonly used forms of SPPS -- Fmoc and Boc. Unlike ribosome protein synthesis, solid-phase peptide synthesis proceeds in a C-terminal to N-terminal fashion. The N-termini of amino acid monomers is protected by these two groups and added onto a deprotected amino acid chain.

Automated synthesizers are available for both techniques, though many research groups continue to perform SPPS manually.

Boc SPPS: SPPS was invented according to the t-Boc ((tert)-(B)utyl (o)xy (c)arbonyl) method. To remove Boc from a growing peptide chain, acidic conditions are used (usually neat TFA). Removal of side-chain protecting groups and the peptide from the resin at the end of the synthesis is achieved by incubating in hydrofluoric acid.

Fmoc SPPS: Fmoc stands for (F)luorenyl-(m)eth(o)xy-(c)arbonyl which describes the Fmoc protecting group. To remove an Fmoc from a growing peptide chain, basic conditions (usually 20% piperidine in DMF) are used. Removal of side-chain protecting groups and peptide from the resin is achieved by incubating in trifluoroacetic acid (TFA).

Solid supports: The physical properties of the solid support, and the applications to which it can be utilized, vary with the material from which the support is constructed, the amount of crosslinking, as well as the linker and handle being used. An example of a support is an H-Cys(Trt)-2-ClTrt resin.

*Polystyrene resin:* This is a versatile resin, which is quite useful in multi-well, automated peptide synthesis, due to its minimal swelling in DCM.

*Polyamide resin:* This is a useful and versatile resin. It can swell more than polystyrene, in which case it may not be suitable for some automated synthesizers.

Examples of types of resins include: Alkenyl Resins, Amine-Functionalized Resins, BHA Resins, Br-Functionalized Resins, Chloromethyl Resins, CHO-Functionalized Resins, Cl-Functionalized Resins, COOH-Functionalized Resins, HYPOGEL® Resins, I-Functionalized Resins, JANDAJELS™, MBHA Resins, NH2-Functionalized Resins, Nitrophenyl Carbonates, OH-Functionalized Resins, Oxime Resins, Peg Cross-Linked, Boc-/Bzl Peptide Synthesis, Fmoc-/tBu Peptide Synthesis, Phoxime Resin, Resin Matrices, Rink Acid Resins, Rink Resins, Safety Catch Resins, TENTAGEL® Resins, Thiol-Functionalized Resins, Triazine-Based Resins, Trityl Amines, Wang Resins, Wang Derivatives. Numerous resins of each type are available and know in the art.

Protective groups: Due to amino acid excesses used to ensure complete coupling during each synthesis step, polymerization of amino acids is common in reactions where each amino acid is not protected. In order to prevent this polymerization, protective groups are used. This adds additional deprotection phases to the synthesis reaction, creating a repeating design flow as follows:
Protective group is removed from trailing amino acids in a deprotection reaction
   - Deprotection reagents washed away to provide clean coupling environment;
   - Protected amino acids dissolved in a solvent such as dimethylformamide (DMF) are combined with coupling reagents are pumped through the synthesis column; and
   - Coupling reagents washed away to provide clean deprotection environment.

*Protective Groups:* Currently, two protective groups (Fmoc, Boc) are commonly used in solid-phase peptide synthesis. Their lability is caused by the carbamate group which readily releases CO₂ for an irreversible decoupling step.

*Fmoc protective group:* Fmoc (9-fluorenylmethyl carbamate) is currently a widely used protective group that is generally removed from the N terminus of a peptide in the iterative synthesis of a peptide from amino acid units. The advantage of Fmoc is that it is cleaved under very mild basic conditions (e.g., piperidine), but stable under acidic conditions. This allows mild acid labile protecting groups that are stable under basic conditions, such as Boc and benzyl groups, to be used on the side-chains of amino acid residues of the target peptide. This orthogonal protecting group strategy is common in the art of organic synthesis.

*Boc protective group:* Before the Fmoc group became popular, the Boc group was commonly used for protecting the terminal amine of the peptide, requiring the use of more acid stable groups for side chain protection in orthogonal strategies. It retains usefulness in reducing aggregation of peptides during synthesis. Boc groups can be added to amino acids with boc anhydride and a suitable base.

*Benzyloxy-carbonyl (Z) group:* Another carbamate based group is the benzyloxy-carbonyl (Z) group. It is removed in harsher conditions: HBr/acetic acid or catalytic hydrogenation. It can be used for side chain protection.

*Alloc protecting group:* The allyloxycarbonyl (alloc) protecting group is often used to protect a carboxylic acid, hydroxyl, or amino group when an orthagonal deprotection scheme is required. It is sometimes used when conducting on-resin cyclic peptide formation, where the peptide is linked to the resin by a side-chain functional group. The alloc group can be removed using tetrakis(triphenylphosphine)palladium(0) along with a 37:2:1 mixture of chloroform, acetic acid, and N-methylmorpholine (NMM) for 2 hours. The resin must then be carefully washed 0.5 % DIPEA in DMF, 3x10 ml of 0.5 % sodium diethylthiocarbamate in DMF, and then 5x10 ml of 1:1 DCM:DMF.

*Lithographic protecting groups:* For special applications, like protein microarrays, lithographic protecting groups are used. Those groups can be removed through exposure to light.

Activating Group: For coupling the peptides the carboxyl group is usually activated. This is important for speeding up the reaction. There are two main types of activating groups: carbodiimides and aromatic oximes.

*Carbodiimides:* Most common are dicyclohexylcarbodiimide (DCC) and diisopropylcarbodiimide (DIC). Reaction with a carboxylic acid yields a highly reactive O-acyl-urea. During artificial protein synthesis (such as Fmoc solid-state synthesizers), the C-terminus is often used as the attachement site on which the amino acid monomers are added. To enhance the electrophilicity of carboxylate group, the negatively charged oxygen must first be "activated" into a better leaving group. DCC is used for this purpose. The negatively charged oxygen will act as a nucleophile, attacking the central carbon in DCC. DCC is temporarily attached to the former carboxylate group (which is now an ester group), making nucleophilic attack by an amino group (on the attaching amino acid) to the former C-terminus(carbonil group) more efficient.

*Aromatic oximes:* Examples inlcude 1-hydroxy-benzotriazole (HOBt) and 1-hydroxy-7-aza-benzotriazole (HOAt). These substances can react with the O-acylurea to form an active ester which is less reactive and less in danger of racemization.

Newer developments omit the carbodiimides totally. The active ester is introduced as a uronium or phosphonium salt of a non-nucleophilic anion (tetrafluoroborate or hexafluorophosphate): HBTU, HATU, PyBOP.

Synthesizing long peptides: Stepwise elongation, in which the amino acids are connected step-by-step in turn, is ideal for small peptides containing between 2 and 100 amino acid residues. Another method is fragment condensation, in which peptide fragments are coupled. Although the former can elongate the peptide chain without racemization, the yield drops if only it is used in the creation of long or highly polar peptides. Fragment condensation is better than stepwise elongation for synthesizing sophisticated long peptides, but its use must be restricted in order to protect against racemization. Fragment condensation is also undesirable since the coupled fragment must be in gross excess, which may be a limitation depending on the length of the fragment.

Chemical ligation can be used: Unprotected peptide chains react chemoselectively in aqueous solution. A first kinetically controlled product rearranges to form the amide bond. The most common form native chemical ligation uses a peptide thioester that reacts with a terminal cysteine residue.

### Pharmaceutical Compositions

A peptide of the disclosure can be formulated as a pharmaceutical composition, e.g., for administration to a subject to treat pain. The peptide can be administered alone or in combination with another therapy for pain, either in the same composition or as a separate composition.

Typically, a pharmaceutical composition includes a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible. The composition can include a pharmaceutically acceptable salt, e.g., an acid addition salt or a base addition salt (see e.g., Berge, S.M., et al. (1977) J. Pharm. Sci. 66:1-19).

Pharmaceutical formulation is a well-established art, and is further described, e.g., in Gennaro (ed.), Remington: The Science and Practice of Pharmacy, 20th ed., Lippincott, Williams & Wilkins (2000) (ISBN: 0683306472); Ansel et al., Pharmaceutical Dosage Forms and Drug Delivery Systems, 7th Ed., Lippincott Williams & Wilkins Publishers (1999) (ISBN: 0683305727); and Kibbe (ed.), Handbook of Pharmaceutical Excipients American Pharmaceutical Association, 3rd ed. (2000) (ISBN: 091733096X).

In one embodiment, the peptide is formulated with excipient materials, such as saline, sodium chloride, sodium dibasic phosphate heptahydrate, sodium monobasic phosphate, and a stabilizer. It can be provided, for example, in a buffered solution at a suitable concentration and can be stored at 2-8°C, or at about -20°C.

The pharmaceutical compositions may be in a variety of forms. These include, for example, liquid, semi-solid and solid dosage forms, such as liquid solutions (e.g., injectable and infusible solutions), dispersions or suspensions, tablets, pills, powders, liposomes, and suppositories. The preferred form can depend on the intended mode of administration and therapeutic application.

The compositions can be administered by a parenteral mode (e.g., intravenous, subcutaneous, intraperitoneal, or intramuscular injection). The phrases "parenteral administration" and "administered parenterally" as used herein mean modes of administration other than enteral and topical administration, usually by injection, and include, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural, and intrasternal injection and infusion.

The compositions can be administered by an enteral route, e.g., via the digestive tract, e.g., by oral administration. For example, the composition may be administered as a tablet, a capsule, a caplet, a pill, a powder, a drop, a suspension, a solution, a paste, a gel, or other oral dosage form. The enteral route includes administration by gastric feeding tube, duodenal feeding tube, or gastrostomy, or rectally, e.g., for a composition in suppository or enema form.

The compositions can be administered for topical administration, e.g., at a site of pain. Topical administration includes, for example, epicutaneous, intranasal, inhalational, and vaginal administration. The composition can be administered to skin (e.g., for a burn, blister, or cut), lip, gum, tooth, oral cavity, eye, ear, nail bed, or throat, etc., e.g., at the site of pain. The composition for topical administration can be in a cream, gel, lotion, or salve, etc.

The composition can be formulated as a solution, microemulsion, dispersion, liposome, or other ordered structure suitable for stable storage at high concentration. Sterile injectable solutions can be prepared by incorporating a peptide described herein in the required amount in an appropriate solvent with one or a combination of ingredients enumerated above, as required, followed by filtered sterilization. Generally, dispersions are prepared by incorporating a peptide described herein into a sterile vehicle that contains a basic dispersion medium and the required other ingredients from those enumerated above. In the case of sterile powders for the preparation of sterile injectable solutions, the preferred methods of preparation are vacuum drying and freeze-drying that yield a powder of a peptide described herein plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption (e.g., for controlled release formulations), for example, monostearate salts and gelatin.

In certain embodiments, the peptide may be prepared with a carrier that will protect the peptide against rapid release (e.g., to prepare a controlled release formulation), including implants, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known. See, e.g., Sustained and Controlled Release Drug Delivery Systems, J.R. Robinson, ed., Marcel Dekker, Inc., New York, 1978.

The peptide can be modified, e.g., with a moiety that improves its stabilization and/or retention in circulation, e.g., in blood, serum, or other tissues, e.g., by at least 1.5, 2, 5, 10, or 50 fold.

For example, the peptide can be associated with (e.g., conjugated to) a polymer, e.g., a substantially non-antigenic polymer, such as a polyalkylene oxide or a polyethylene oxide. Suitable polymers will vary substantially by weight. Polymers having molecular number average weights ranging from about 200 to about 35,000 Daltons (or about 1,000 to about 15,000, and 2,000 to about 12,500) can be used.

For example, a peptide can be conjugated to a water soluble polymer, e.g., a hydrophilic polyvinyl polymer, e.g., polyvinylalcohol or polyvinylpyrrolidone. A non-limiting list of such polymers includes polyalkylene oxide homopolymers such as polyethylene glycol (PEG) or polypropylene glycols, polyoxyethylenated polyols, copolymers thereof and block copolymers thereof, provided that the water solubility of the block copolymers is maintained. Additional useful polymers include polyoxyalkylenes such as polyoxyethylene, polyoxypropylene, and block copolymers of polyoxyethylene and polyoxypropylene; polymethacrylates; carbomers; and branched or unbranched polysaccharides.

When the peptide is used in combination with a second agent (e.g., another agent described herein), the two agents can be formulated separately or together. For example, the respective pharmaceutical compositions can be mixed, e.g., just prior to administration, and administered together or can be administered separately, e.g., at the same or different times.

Other therapeutic agents described herein can also be provided as pharmaceutical composition, e.g., by standard methods or method described herein.

### Administration

The peptide can be administered to a subject, e.g., a human subject, by a variety of methods. For many applications, the route of administration is parenteral, e.g., one of: intravenous injection or infusion (IV), intraarterial injection, subcutaneous injection (SC), intraperitoneally (IP), intracardiac injection, intraosseous infusion, intradermal injection, intraperitoneal infusion or injection, intravitreal injection, intramuscular injection, intrathecal injection, intra-articular injection, or epidural administration. It is also possible to use. In some preferred embodiments, the peptide is administered by an enteral route (e.g., orally). The peptide can be administered locally, e.g., topically (e.g., epicutaneously, intranasally, inhalationally, vaginally, etc.) (e.g., in a cream, gel, lotion, or salve), e.g., to the skin or lip or gum or oral cavity or throat, e.g., at the site of pain. In some cases, administration may be directly to a site of pain.

The peptide can be administered locally or systemically.

The peptide can be administered e.g., by injection, infusion, diffusion, implants, topical application, or oral delivery.

The blocking agent can be administered as a fixed dose, or in a µg/kg or mg/kg dose.

The dose can also be chosen to reduce or avoid production of antibodies against the peptide.

The route and/or mode of administration of the peptide can also be tailored for the individual case, e.g., by evaluating or monitoring the subject, e.g., using electromyography (EMG), nerve conduction studies, evoked potential (EP) studies, magnetic resonance imaging (MRI), neurological examination, X-rays, and/or standard parameters associated with the particular disorder, e.g., criteria for assessing back pain.

Dosage regimens are adjusted to provide the desired response, e.g., a therapeutic response or a combinatorial therapeutic effect. Generall y, any combination of doses (either separate or co-formulated) of the peptide (and optionally a second agent, e.g., as described herein) can be used in order to provide a subject with the peptide in bioavailable quantities. For example, doses in the range of 0.1 µg/kg -10 mg/kg, 1 µg/kg- 1 mg/kg, 1 µg/kg- 100 µg/kg, 5 µg/kg- 500 µg/kg, 0.1-100 mg/kg, 0.5-100 mg/kg, 1 mg/kg -100 mg/kg, 0.5-20 mg/kg, or 1-10 mg/kg can be administered. Other doses can also be used.

Dosage unit form or "fixed dose" as used herein refers to physically discrete units suited as unitary dosages for the subjects to be treated; each unit contains a predetermined quantity of active compound calculated to produce the desired therapeutic effect in association with the required pharmaceutical carrier and optionally in association with the other agent. Single or multiple dosages may be given. Alternatively, or in addition, the peptide may be administered via continuous infusion.

The peptide can be administered, e.g., once or twice daily, or about one to four times per week, or preferably weekly, biweekly, or monthly, e.g., for between about 1 to 10 weeks, or longer if needed, e.g., for the treatment of chronic pain or for a subject undergoing a long course of treatment, e.g., a subject undergoing chemotherapy. The skilled artisan will appreciate that certain factors may influence the dosage and timing required to effectively treat a subject, including but not limited to the severity of the disease or disorder, formulation, route of delivery, previous treatments, the general health and/or age of the subject, other diseases present, and other treatments a subject has undergone. Moreover, treatment of a subject with a therapeutically effective amount of a peptide can include a single treatment or, preferably, can include a series of treatments. Animal models can also be used to determine a useful dose, e.g., an initial dose or a regimen. For example, animal studies can be used to measure how long the analgesic effect of a peptide lasts.

If a subject is at risk for experiencing pain (e.g., the subject has scheduled a surgical procedure), the peptide can be administered before the event that may cause pain (e.g., surgery), e.g., as a preventative measure. The duration of such preventative treatment can be a single dosage of the blocking agent or the treatment may continue (e.g., multiple dosages) from a time before the event, during the event, and/or after the event, e.g., to minimize the pain experienced by the subject. For example, a subject at risk for experiencing pain may be treated with the blocking agent hours or days before the event that may cause pain, so as to prevent the pain from occurring or to decrease the amount of pain experienced.

A pharmaceutical composition may include a "therapeutically effective amount" of a peptide described herein. Such effective amounts can be determined based on the effect of the administered agent (e.g., peptide), or the combinatorial effect of agents if more than one agent is used. A therapeutically effective amount of an agent may also vary according to factors such as the type of pain, disease state, age, sex, and weight of the subject, and the ability of the agent to elicit a desired response in the subject, e.g., amelioration of pain. A therapeutically effective amount is also one in which any toxic or detrimental effects of the composition are outweighed by the therapeutically beneficial effects.

As used herein, the "subject" can be any organism that experiences pain, e.g., a mammal, e.g., human, farm animal (e.g., horse, donkey, mule, cattle, cow, bull, sheep, pig, etc.), domestic pet (e.g., dog, cat, rat, mouse, rabbit, hamster, guinea pig, ferret, etc.), or animal kept in a zoological park or aquarium (e.g., giraffe, lion, tiger, bear, zebra, monkey, gorilla, whale, dolphin, shark, etc.).

### Devices and Kits for Therapy

Pharmaceutical compositions that include the peptide can be administered with a medical device. The device can include, e.g., one or more housings for storing pharmaceutical preparations that include the peptide, and can be configured to deliver one or more unit doses of the peptide. The device can be further configured to administer a second agent, e.g., a second pain treatment agent described herein, either as a single pharmaceutical composition that also includes the peptide or as two separate pharmaceutical compositions.

For example, the pharmaceutical composition can be administered with a needleless hypodermic injection device, such as the devices disclosed in U.S. Pat. Nos. 5,399,163; 5,383,851; 5,312,335; 5,064,413; 4,941,880; 4,790,824; or 4,596,556. Examples of well-known implants and modules include: U.S. Pat. No. 4,487,603, which discloses an implantable micro-infusion pump for dispensing medication at a controlled rate; U.S. Pat. No. 4,486,194, which discloses a therapeutic device for administering agents through the skin; U.S. Pat. No. 4,447,233, which discloses a medication infusion pump for delivering medication at a precise infusion rate; U.S. 4,447,224, which discloses a variable flow implantable infusion apparatus for continuous drug delivery; U.S. Pat. No. 4,439,196, which discloses an osmotic drug delivery system having multi-chamber compartments; and U.S. Pat. No. 4,475,196, which discloses an osmotic drug delivery system. Many other devices, implants, delivery systems, and modules are also known.

A peptide can be provided in a kit. In one embodiment, the kit includes (a) a container that contains a composition that includes a peptide described herein, and optionally (b) informational material. The informational material can be descriptive, instructional, marketing or other material that relates to the methods described herein and/or the use of the peptide for therapeutic benefit.

In an embodiment, the kit also includes a second agent for treating pain, e..g, another agent described herein. For example, the kit includes a first container that contains a composition that includes the peptide, a second container that includes the second agent, and optionally informational material. The informational material can be descriptive, instructional, marketing or other material that relates to the methods described herein and/or the use of the agents for therapeutic benefit.

The informational material of the kits is not limited in its form. In one embodiment, the informational material can include information about production of the compound (e.g., peptide), molecular weight of the compound, concentration, date of expiration, batch or production site information, and so forth. In one embodiment, the informational material relates to methods of administering the peptide and/or second agent, e.g., in a suitable dose, dosage form, or mode of administration (e.g., a dose, dosage form, or mode of administration described herein), to treat a subject who is experiencing or who is at risk for experiencing pain. The information can be provided in a variety of formats, include printed text, computer readable material, video recording, or audio recording, or information that provides a link or address to substantive material.

In addition to the peptide, the composition in the kit can include other ingredients, such as a solvent or buffer, a stabilizer, or a preservative. The peptide can be provided in any form, e.g., liquid, dried or lyophilized form, preferably substantially pure and/or sterile. When the agents are provided in a liquid solution, the liquid solution preferably is an aqueous solution. When the agents are provided as a dried form, reconstitution generally is by the addition of a suitable solvent. The solvent, e.g., sterile water or buffer, can optionally be provided in the kit.

The kit can include one or more containers for the composition or compositions containing the agents (e.g., the peptide or the peptide in combination with another agent). In some embodiments, the kit contains separate containers, dividers or compartments for the composition and informational material. For example, the composition can be contained in a bottle, vial, or syringe, and the informational material can be contained in a plastic sleeve or packet. In other embodiments, the separate elements of the kit are contained within a single, undivided container. For example, the composition is contained in a bottle, vial, or syringe that has attached thereto the informational material in the form of a label. In some embodiments, the kit includes a plurality (e.g., a pack) of individual containers, each containing one or more unit dosage forms (e.g., a dosage form described herein) of the composition. For example, the kit includes a plurality of oral dosage forms, syringes, ampules, foil packets, blister packs, or medical devices, e.g., each containing a single unit dose of the composition containing the peptide. The containers can include a combination unit dosage, e.g., a unit that includes both the peptide and the second agent, e.g., in a desired ratio. For example, the kit includes a plurality of oral dosage forms, syringes, ampules, foil packets, blister packs, or medical devices, e.g., each containing a single combination unit dose. The containers of the kits can be air tight, waterproof (e.g., impermeable to changes in moisture or evaporation), and/or light-tight.

The kit optionally includes a device suitable for administration of the composition, e.g., a syringe or other suitable delivery device. The device can be provided preloaded with one or both of the agents or can be empty, but suitable for loading.

### EXAMPLES

### Example 1

For Examples 2-6 provided herein, the 7A14A peptide was chemically synthesized by a stepwise solid-phase synthesis method using N-9- fluorenylmethoxycarbonyl (Fmoc) chemistry with Fmoc-Ala-NOVASYN® TGA resin (Nova Biochem.) on a Shimadzu PSSM-8 peptide synthesizer (Shimadzu Corp., Kyoto, Japan). All Fmoc-L-amino acids were purchased from Nova Biochem. Cleavage of the peptide from the resin was achieved by treatment with a mixture of TFA/anisole/1,2-ethanedithiol (94:5:1, by volume) at room temperature for 2 hours. After removal of the resin by filtration and washing twice with TFA, the combined filtrate was added drop-wise to diethyl ether at 0°C and then centrifuged at 3000rpm for 10 min. to collect the precipitate of crude peptide. The obtained crude synthetic peptide was purified by reverse-phase HPLC using YMC-Pack ODS, 20 x 150 mm (Yamamura Kagaku, Kyoto, Japan) with isocratic elution of 15% CH₃CN/H₂O/0.1% TFA at a flow rate of 7 ml min. The homogeneity and the sequence was confirmed by MALDI-TOF MS and HPLC.

### Example 2

The anti-nociceptive effect of the 7A14A peptide (SEQ ID NO:2) was tested in a prostaglandin E2 (PGE2)-induced hyperalgesia pain model. The peptide was diluted in saline to a concentration of 5 µg/kg and orally administered to rats. Animals that received saline through the same route were used as controls.

For the induction of hyperalgesia, a stock solution of prostaglandin E2 (PGE2) was prepared by dissolving 500 µg of PGE2 in 1 ml of ethanol. At the moment of use, this stock solution was re-diluted in sterile saline. The dose of prostaglandin used was 100 ng in 100 µl of saline, administered by the intraplantar route per paw (100 ng/paw).

To evaluate pain sensitivity, a rat paw pressure test was used (Analgesy-Meter Ugo Basile®, Italy), performed according to the method described by Randall & Selitto (Randall L.O. and Selitto J.J. Arch. Intern. Pharmacodyn. 111:209-219, 1957). In this test, a force in grams (g), with increasing magnitude (16 g/s), is continuously applied on the dorsal surface of one of the hind paws of the rat and interrupted when the animal reacts by "withdrawing" the paw. In this model, the pain threshold (the limit of pain) is represented as the force (g) necessary to induce the withdrawal of the paw. The limit of pain was measured before and 3 hours after the intraplantar injection of PGE2 (100 ng/paw). The peptide or saline (control) were orally administered to the animals immediately before the PGE2 was administered. (See also WO 2005/107357).

The results of the experiment are shown in FIG.1 (MI: initial measurement before PGE2 injection; MF: final measurement after PGE2 injection). The data represent the average ± e.p.m. of 5 animals per group. *p<0.05 by comparison with the initial measurement and **p<0.05 for comparison with the control group. As shown in the figure, the orally administered peptide decreases sensitivity of rat paws to pressure in a model of hyperalgesia and has an analgesic effect.

### Example 3

The duration of the anti-nociceptive effect caused by the 7A14A peptide was evaluated in the PGE2-induced hyperalgesia model. For evaluation of pain sensitivity, the rat paw pressure test was used. The experiment was performed as described above.

The results of the experiment are shown in FIG.2. The pain threshold (limit of pain), represented by the force (in grams) necessary for withdrawal of the paw, was determined before (time 0) and 3, 120, and 192 hours (final measure) after oral treatment with the peptide (5 µg/kg) or saline (control group). PGE2 (100 ng/paw) was injected 3 hours before each final measure. The data represent the average ± e.p.m. of 6 animals per group. *p<0.05 for comparison with the control group. As these results demonstrate, the anti-nociceptive effect of the peptide is present 192 hours after oral administration of the peptide.

### Example 4

The stability of the 7A14A peptide after dilution and after different durations of storage of the diluted peptide was evaluated. The peptide was diluted with saline to give a final concentration of 5 µg/kg. The diluted peptide was then stored at -20°C for 0, 30, 40, 60, or 90 days prior to use in a hyperalgesia model.

The diluted peptide was evaluated in the PGE2-induced hyperalgesia model. The experiment was performed as described above.

For the evaluation of pain sensitivity, a rat paw pressure test was utilized. The pain threshold (limit of pain), represented by the force (in grams) necessary for withdrawal of the paw, was determined 3 hours after the intraplantar injection of PGE2 (100 ng/paw). The peptide (5 µg/kg), diluted at the moment of use or after 30, 40, 60 or 90 days (dias) of storage after dilution, or saline (salina; control group) were orally administered immediately before administration of the PGE2.

The results are shown in FIG. 3. The data represent the average ± e.p.m. of 5 animals per group. *p<0.05 for comparison with the initial measure, and **p<0.05 for comparison with the control group. As shown in the figure, the peptide in diluted form was stable and able to cause an anti-nociceptive effect and analgesia after 90 days of storage at-20°C.

### Example 5

The duration of the anti-nociceptive effect caused by the 7A14A peptide was evaluated in a model in which the hyperalgesia was induced by chronic constriction of the rat sciatic nerve, which is a model of persistent neuropathic pain. For the induction of neuropathic pain, surgery was performed in the sciatic nerve in accordance with the method described by Bennett, G.J. and Xie, Y.K. (Pain, 33:87-107, 1988). Briefly, the surgery was performed as follows. The animals were anesthetized with halothane. The sciatic nerve was exposed in the middle region of the thigh, moving away the femoral biceps muscle. Close to the trifurcation of the sciatic nerve, 7 mm of distance from the trifurcation, 4 loose ligations were performed (chromed catgut 4-0) around it, distant from each other in approximately 1mm. The bindings were performed along the nerve, up to 4-5mm from the initial point. The incision was sutured in layers, using silk suture thread number 4-0.

For evaluation of pain sensitivity, the rat paw pressure test was used, as described above. The peptide (at a dose of 1 µg/kg) or the saline were orally administered to the rats on day 14 after surgery. The paw pressure test was applied before surgery (MI: initial measurement), and on day 14 after surgery, before any pharmacological treatment, and at 24, 48, 72 and 96 hours after oral administration of saline or peptide. The results are shown in FIG. 4. As shown in the figure, the orally administered peptide decreases sensitivity of to pressure in this model of chronic hyperalgesia, showing an analgesic effect in neuropathic pain that lasts for 72 hours. *p<0.05 and **p<0.05.

### Example 6

The anti-nociceptive effects of the ENPAK peptide (SEQ ID NO:3) and 7A14A peptide (SEQ ID NO:2) was tested in an acetic acid-induced abdominal writhing assay, which is a model for visceral pain. The peptide was diluted in saline to a concentration indicated in FIG. 5A and 5B and orally administered to mice. Animals that received saline through the same route were used as controls.

The mouse writhing test was based on the method of Koster et al. (1959). For induction of writhing, mice treated 40 minutes with morphine (4.5mg/kg, *i.p.*), mice treated 100 minutes with ENPAK (1, 6, 9, 12.5, 25, 50, 75, 125, 250 µg/kg, oral), mice treated 100 minutes with 7A14A (12.5, 25, 50, 75, 125, 250, 500, 750 µg/kg, oral), and control mice; were injected with a dose of 60mg/kg acetic acid by intraperitoneal route. The abdominal contortions, consisting of contractions of the abdominal muscles and a stretching of hind limbs, were counted cumulatively over a period of 20 minutes after injection. The anti-nociceptive activity was observed as a reduction in the number of abdominal contortions between peptide-treated and non-treated animals.

Data represents mean values ± e.p.m. of 4-34 animals per group. *Significantly different from mean values of Saline group (* p<O.05); ** Significantly different from mean values of Morphine group (** p<0.05).

## Claims

1. An isolated peptide that comprises an amino acid sequence that is at least 90% identical to the amino acid sequence: pGlu-Phe-Ser-Pro-Glu-Asn-Ala-Gln-Gly-Glu-Ser-Gln-Pro-Ala (SEQ ID NO: 2), with the proviso that the peptide does not comprise the amino acid sequence SEQ ID NO: 3 or SEQ ID NO: 5 and wherein amino acid residues R7 and R14 are not linked by an intramolecular disulfide bridge.

2. The peptide of claim 1, wherein the peptide comprises the amino acid sequence SEQ ID NO: 2.

3. A pharmaceutical composition comprising the peptide of claim 1 or 2.

4. The pharmaceutical composition according to claim 3 for use for treating or preventing painful conditions or conditions mediated by opioid receptors in a subject.

5. The pharmaceutical composition for use according to claim 4, wherein the peptide comprised in said pharmaceutical composition is a direct or indirect opioid receptor agonist.

6. The pharmaceutical composition according to claim 3 for use for treating or preventing pain in a subject.

7. The pharmaceutical composition according to claim 3 for use for causing analgesia in a subject.

8. A process for producing a peptide comprising SEQ ID NO: 2, wherein the process comprises peptide synthesis in solid phase.

## Patentansprüche

1. Isoliertes Peptid, umfassend eine Aminosäuresequenz, die wenigstens zu 90 % identisch mit der Aminosäuresequenz: pGlu-Phe-Ser-Pro-Glu-Asn-Ala-Gln-Gly-Glu-Ser-Gln-Pro-Ala (SEQ ID NO: 2) identisch ist, unter der Bedingung, dass das Peptid die Aminosäuresequenz SEQ ID NO: 3 oder SEQ ID NO: 5 nicht enthält und wobei die Aminosäurereste R7 und R14 nicht durch eine intramolekulare Disulfidbrücke verknüpft sind.

2. Peptid nach Anspruch 1, wobei das Peptid die Aminosäuresequenz SEQ ID NO: 2 umfasst.

3. Pharmazeutische Zusammensetzung, umfassend das Peptid nach Anspruch 1 oder 2.

4. Pharmazeutische Zusammensetzung nach Anspruch 3 zur Verwendung zur Behandlung oder Verhütung von schmerzhaften Zuständen oder Zuständen, die durch Opioidrezeptoren in einem Probanden vermittelt werden.

5. Pharmazeutische Zusammensetzung zur Verwendung nach Anspruch 4, wobei das in der genannten pharmazeutischen Zusammensetzung enthaltene Peptid ein direkter oder indirekter Opioidrezeptoragonist ist.

6. Pharmazeutische Zusammensetzung nach Anspruch 3 zur Verwendung zur Behandlung oder Verhütung von Schmerzen in einem Probanden.

7. Pharmazeutische Zusammensetzung nach Anspruch 3 zur Verwendung bei dem Verursachen einer Analgesie in einem Probanden.

8. Verfahren zur Herstellung eines Peptids umfassend SEQ ID NO: 2, wobei das Verfahren eine Peptidsynthese in fester Phase umfasst.

## Revendications

1. Peptide isolé qui comprend une séquence d'acides aminés qui est au moins 90 % identique à la séquence d'acides aminés : pGlu-Phe-Ser-Pro-Glu-Asn-Ala-Gln-Gly-Glu-Ser-Gln-Pro-Ala (SEQ ID NO: 2), à condition que le peptide ne comprenne pas la séquence d'acides aminés SEQ ID NO: 3 ou SEQ ID NO: 5, et où les résidus d'acides aminés R7 et R14 ne sont pas liés par un pont disulfure intramoléculaire.

2. Peptide selon la revendication 1, où le peptide comprend la séquence d'acides aminés SEQ ID NO: 2.

3. Composition pharmaceutique comprenant le peptide selon la revendication 1 ou 2.

4. Composition pharmaceutique selon la revendication 3 pour son utilisation dans le traitement ou la prévention d'affection douloureuses ou d'affections médiées par les récepteurs opioïdes chez un sujet.

5. Composition pharmaceutique pour son utilisation selon la revendication 4, où le peptide compris dans ladite composition pharmaceutique est un agoniste direct ou indirect des récepteurs opioïdes.

6. Composition pharmaceutique selon la revendication 3 pour son utilisation dans le traitement ou la prévention de la douleur chez un sujet.

7. Composition pharmaceutique selon la revendication 3 pour son utilisation pour provoquer une analgésie chez un sujet.

8. Procédé de production d'un peptide comprenant SEQ ID NO:2, où le procédé comprend une synthèse peptidique en phase solide.


### SEQUENCE LISTING

<110> COINFAR-CONSÓRCIO DE INDÚSTRIAS FARMACÊUTICAS
<120> ANALGESIC COMPOUNDS
<130> 1331-0030
<150> US 60/915,247
   <151> 2007-05-01
<160> 4
<170> FastSEQ for Windows Version 4.0
<210> 1
   <211> 14
   <212> PRT
   <213> Artificial Sequence
<220>
   <223> Synthetically generated peptide
<220>
   <221> VARIANT
   <222> 1
   <223> Xaa = pyroglutamate
<220>
   <221> VARIANT
   <222> 2
   <223> Xaa = Phe, Trp, Tyr, Leu or Thr
<220>
   <221> VARIANT
   <222> 4
   <223> Xaa = Pro or Arg
<220>
   <221> VARIANT
   <222> 5
   <223> Xaa = Glx or Asx or Gly
<220>
   <221> VARIANT
   <222> 6
   <223> Xaa = Asn, Gln or Leu
<220>
   <221> VARIANT
   <222> 7..
   <223> Xaa = is any amino acid
<220>
   <221> VARIANT
   <222> 8
   <223> Xaa = is any amino acid except Lys
<220>
   <221> VARIANT
   <222> 10
   <223> Xaa = Glx or Asx
<220>
   <221> VARIANT
   <222> 12
   <223> Xaa = Glx or Lys
<220>
   <221> VARIANT
   <222> 14
   <223> Xaa = any amino acid
<400> 1
   Xaa Xaa Ser Xaa Xaa Xaa Xaa Xaa Gly Xaa Ser Xaa Pro Xaa
    1               5                  10
<210> 2
   <211> 14
   <212> PRT
   <213> Artificial Sequence
<220>
   <223> Synthetically generated peptide
<400> 2
   Glu Phe Ser Pro Glu Asn Ala Gln Gly Glu Ser Gln Pro Ala
   1 5 10
<210> 3
   <211> 14
   <212> PRT
   <213> Artificial Sequence
<220>
   <223> Synthetically generated peptide
<400> 3
   Glu Phe Ser Pro Glu Asn Cys Gln Gly Glu Ser Gln Pro Cys
   1 5 10
<210> 4
   <211> 14
   <212> PRT
   <213> Artificial Sequence
<220>
   <223> Synthetically generated peptide
<220>
   <221> VARIANT
   <222> 1
   <223> Xaa = pyroglutamate
<220>
   <221> VARIANT
   <222> 2
   <223> Xaa = Phe, Trp, Tyr, Leu or Thr
<220>
   <221> VARIANT
   <222> 4
   <223> Xaa = Pro or Arg
<220>
   <221> VARIANT
   <222> 5
   <223> Xaa = Glx or Asx or Gly
<220>
   <221> VARIANT
   <222> 6
   <223> Xaa = Asn, Gin or Leu
<220>
   <221> VARIANT
   <222> 8
   <223> Xaa = is any amino acid except Lys
<220>
   <221> VARIANT
   <222> 10
   <223> Xaa = Glx or Asx
<220>
   <221> VARIANT
   <222> 12
   <223> Xaa = Glx or Lys
<400> 4
   Xaa Xaa Ser Xaa Xaa Xaa Cys Xaa Gly Xaa Ser Xaa Pro Cys
    1               5                   10